(19) Europäisches Patentamt
European Patent Office
Office européen des brevets

(11) Numéro de publication: **0 226 511 B1**

(12) **FASCICULE DE BREVET EUROPEEN**

(45) Date de publication du fascicule du brevet: **02.08.89**

(51) Int. Cl.⁴: **C07D 239/06**, C07D 239/04

(21) Numéro de dépôt: **86402703.2**

(22) Date de dépôt: **05.12.86**

(54) **Dérivés de 5-phényl-1,4,5,6-tétrahydropyrimidine, procédé de préparation et utilisation en thérapeutique.**

(30) Priorité: **13.12.85 FR 8518462**
**14.03.86 FR 8603644**

(43) Date de publication de la demande:
**24.06.87 Bulletin 87/26**

(45) Mention de la délivrance du brevet:
**02.08.89 Bulletin 89/31**

(84) Etats contractants désignés:
**AT BE CH DE ES FR GB GR IT LI LU NL SE**

(56) Documents cités:
**TERATOLOGY, vol. 5, no. 3, 1972, pages 287-292; D.G. UPSHALL: "Correlation of chick embryo teratogenicity with the nicotinic activity of a series of tetrahydropyrimidines"**
**CHEMICAL ABSTRACTS, vol. 97, 1982, page 712, résumé no. 162920e, Columbus, Ohio, US; S. KANO et al.: "A new and facile synthesis of 5-arylpyrimidines and 4-arylpyrazoles"**

(73) Titulaire: **LABORATOIRE L. LAFON Société anonyme dite:, 1 rue Georges Médéric, F-94701 Maisons-Alfort(FR)**

(72) Inventeur: **Lafon, Louis, 5, rue de l'Alboni, F-75016 Paris(FR)**

(74) Mandataire: **Clisci, Serge et al, S.A. FEDIT-LORIOT CONSEILS EN PROPRIETE INDUSTRIELLE 38, avenue Hoche, F-75008 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention a trait à des dérivés de 5-phényl-1,4,5,6-tétrahydropyrimidine en tant que produits industriels nouveaux. Elle concerne également le procédé de préparation et l'utilisation en thérapeutique de ces produits notamment en tant qu'agents antidépresseurs du système nerveux central (SNC) et/ou sédatifs.

On sait de l'article de D.G. UPSHALL, Teratology, 5 (No. 3), pages 287-294 (1972), que des dérivés de phényl-1,4,5,6-tétrahydropyrimidine, dans lesquels le groupe phényle est situé en position 2, 3(sic), 4, 5 ou 6 [voir Tableau 1, page 288; et, page 289, colonne de droite, lignes 22-26], ont été préparés et testés sur l'embryon de poulet en vue de leurs éventuelles propriétés tératogènes.

Le Tableau 1 de la page 288 dudit article comprend un grand nombre d'erreurs et fait état de composés que l'on ne peut manifestement pas synthétiser, en particulier les composés Nos. 6, 9 et 21. Parmi les composés susceptibles d'être effectivement préparés, sont mentionnés les 2-phényl- (cf. composé No. 2 qui est présenté comme étant tératogène), 4-phényl- (cf. composé No. 10), 4-(4-chlorophényl)- (cf. composé No. 18), 2-méthyl-4-phényl- (cf. composé No. 22) et 6-phényl- (cf. composé No. 23) 1,4,5,6-tétrahydropyrimidines.

En bref, l'article de D.G. UPSHALL précité ne décrit pas les composés selon l'invention de formule I ci-après, ni leur utilisation en thérapeutique en tant que moyens agissant sur le SNC. Cet article a trait tout au plus à l'absence d'effets tératogènes sur l'embryon de poulet d'un isomère (ledit composé No. 18 sus-visé) d'un produit suivant l'invention (CRL 41 336 objet de l'Exemple 1 ci-après).

Selon l'invention on préconise de nouveaux dérivés de 5-phényl-1,4,5,6-tétrahydropyrimidine qui sont caractérisés en ce qu'ils sont choisis parmi l'ensemble comprenant

(i) les 5-phényl-hydropyrimidines de formule

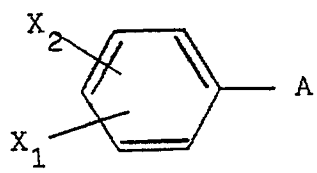

(I)

dans laquelle

$X_1$ et $X_2$, identiques ou différents, représentent chacun H, F, Cl, Br ou $CF_3$, et

A représente un groupe polyhydropyrimidinyle de structure

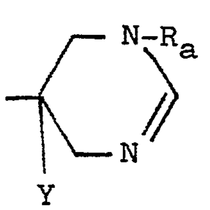 , 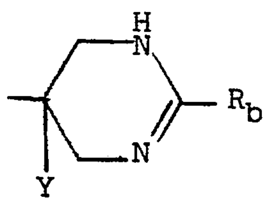 ou 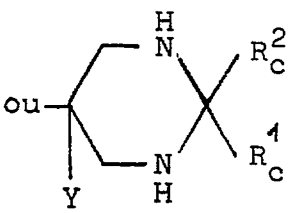

où

Y représente H ou OH,

$R_a$ représente un groupe alkyle en $C_1$-$C_4$ ou un groupe alcanoyle en $C_2$-$C_5$, $R_a$ pouvant représenter l'atome d'hydrogène quand au moins un des groupes $X_1$, $X_2$ et Y est différent de H;

$R_b$ représente un groupe alkyle en $C_1$-$C_3$;

$R_c^1$ et $R_c^2$, identiques ou différents, représentent chacun l'atome d'hydrogène ou un groupe alkyle en $C_1$-$C_3$; et

(ii) leurs sels d'addition.

Sont donc inclus dans cet enseignement

- les 5-phényl-1,4,5,6-tétrahydropyrimides non substituées en position 2 du groupe hydropyrimidinyle de formule

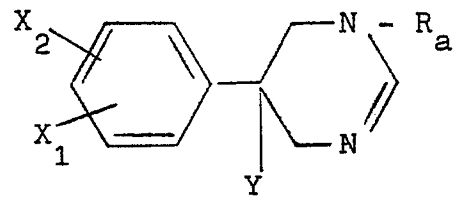

$(I_a)$

(où $X_1$, $X_2$, Y et $R_a$ sont définis comme indiqué ci-dessus dans la formule I) et leurs sels d'addition,
- les 2-alkyl-5-phényl-1,4,5,6-tétrahydropyrimidines de formule

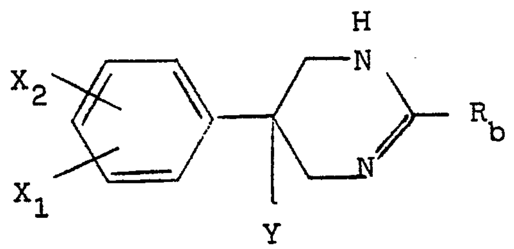

$(I_b)$

(où $X_1$, $X_2$, Y et $R_b$ sont définis comme indiqué ci-dessus dans la formule I) et leurs sels d'addition, et
- les 5-phényl-1,2,3,4,5,6-hexahydropyrimidines de formule

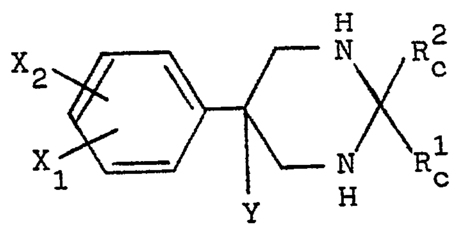

$(I_c)$

(où $X_1$, $X_2$, Y, $R_c^1$ et $R_c^2$ sont définis comme indiqué ci-dessus dans la formule I) et leurs sels d'addition.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier les bases libres de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir les sels d'ammonium, on peut notamment citer $ICH_3$ et $ClCH_3$. D'une manière générale les sels d'acide sont préférés aux sels d'ammonium.

Parmi les groupes alkyle qui interviennent dans la définition du groupe $R_a$ et conviennent selon l'invention, on peut notamment citer les radicaux $CH_3$, $CH_2CH_3$, $CH(CH_3)_2$ et $C(CH_3)_3$, les groupes alkyle préférés étant $CH_3$, $CH_2CH_3$ et $CH(CH_3)_2$.

Parmi les groupes alcanoyle qui interviennent dans la définition du groupe $R_a$ et conviennent selon l'invention, on peut mentionner $COCH_3$, $COCH_2CH_3$, $COCH_2CH(CH_3)_2$, $COCH(CH_3)_2$, $COC(CH_3)_3$ et $COCH_2CH_2CH_3$, le groupe alcanoyle préféré selon l'invention étant $COCH_3$.

Les groupes $X_1$ et $X_2$, qui peuvent être identiques ou différents, représentent chacun l'atome d'hydrogène, un atome d'halogène (tel que F, Cl ou Br) ou le groupe trifluorométhyle, les atomes d'halogène préférés étant F et Cl. De façon pratique quand le noyau phényle est substitué on préfère plus particulièrement que $X_1$ = 3 - $CF_3$ ou (mieux) 4-Cl ou 2-F et que $X_2$ = H.

Comme indiqué ci-dessus, $R_a$ peut représenter l'atome d'hydrogène quand un au moins des $X_1$, $X_2$ et Y est différent de H. De façon avantageuse $R_a$ sera différent de H si, simultanément, (i) Y = H, (ii) $X_2$ = H et (iii) $X_1$ = H ou 4-Cl.

Parmi les groupes $R_b$, $R_c^1$ et $R_c^2$ qui conviennent selon l'invention on peut notamment citer les groupes $CH_3$, $CH_2CH_3$, $CH_2CH_2CH_3$, $CH(CH_3)_2$, le groupe alkyle préféré étant ici $CH_3$. De façon pratique pour les composés 1,2,3,4,5,6-hexahydropyrimidinyle on préfère que $R_c^1$ et $R_c^2$ ne représentent pas l'atome d'hydrogène mais soient plutôt le même groupe alkyle et mieux $CH_3$.

En pratique également on préfère d'une manière générale les composés de formule I dans lesquels un seul des cycles phényle et hydropyrimidinyle est substitué, plutôt que les composés de formule I dans lesquels les deux dits cycles sont chacun substitués.

On a consigné dans les tableaux $I_a$, $I_b$ et $I_c$ ci-après un certain nombre de composés typiques selon l'invention.

TABLEAU $I_a$

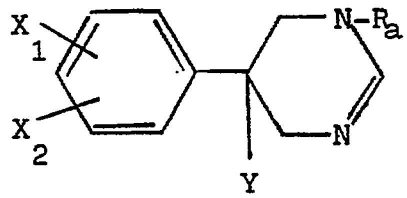

| PRODUITS | No de CODE | $X_1$ | $X_2$ | Y | $R_a$ |
|---|---|---|---|---|---|
| Ex 1(a) | CRL 41 336 | 4-Cl | H | H | H |
| Ex 2(a) | CRL 41 337 | H | H | H | $CH_3$ |
| Ex 3(b) | - | 3-Cl | H | H | H |
| Ex 4(b) | - | 2-Cl | H | H | H |
| Ex 5(a) | - | 4-Cl | H | H | $CH_3$ |
| Ex 6(a) | CRL 41 329 | 2-F | H | H | H |
| Ex 7(a) | CRL 41 378 | H | H | H | $COCH_3$ |
| Ex 8(a) | CRL 41 382 | H | H | OH | H |
| Ex 9(a) | - | 3-Cl | 4-Cl | H | H |
| Ex 10(a) | - | 3-Cl | 4-Cl | OH | H |
| Ex 11(c) | - | 3-Cl | 5-Cl | H | H |
| Ex 12(a) | - | 3-Cl | 5-Cl | H | $CH_3$ |
| Ex 13(a) | - | 2-Br | H | OH | H |
| Ex 14(b) | - | $3-CF_3$ | H | H | H |
| Ex 15(a) | - | $3-CF_3$ | H | H | $CH(CH_3)_2$ |

Notes
(a) : chlorhydrate
(b) : méthanesulfonate
(c) : fumarate

TABLEAU $I_b$

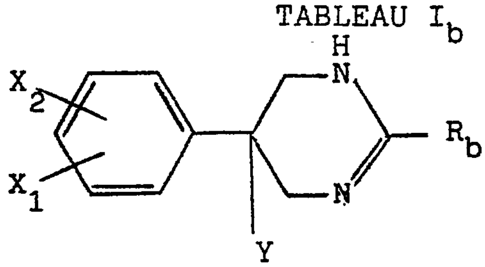

| PRODUITS | No de CODE | $X_1$ | $X_2$ | Y | $R_b$ |
|---|---|---|---|---|---|
| Ex 16 (a) | CRL 41 352 | H | H | H | $CH_3$ |
| Ex 17 (a) | - | 4-Cl | H | H | $CH_3$ |
| Ex 18 (b) | - | 2-F | H | H | $CH_3$ |
| Ex 19 (c) | - | 3-$CF_3$ | H | H | $CH_3$ |
| Ex 20 (b) | - | H | H | OH | $CH_3$ |
| Ex 21 (a) | - | 3-Cl | 4-Cl | OH | $CH_2CH_3$ |

<u>Notes</u>

(a) : chlorhydrate

(b) : méthanesulfonate

(c) : fumarate

TABLEAU $I_c$

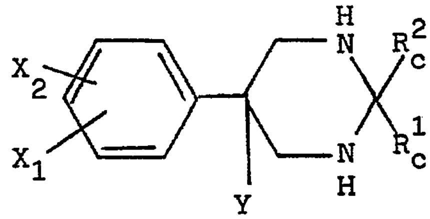

| PRODUIT | No de CODE | $X_1$ | $X_2$ | Y | $R_c^1$ | $R_c^2$ |
|---|---|---|---|---|---|---|
| Ex 22 (a) | CRL 41 330 | 2-F | H | H | $CH_3$ | $CH_3$ |
| Ex 23 (a) | - | H | H | H | $CH_3$ | $CH_3$ |
| Ex 24 (c) | CRL 41 365 | H | H | OH | $CH_3$ | $CH_3$ |
| Ex 25 (a) | - | 4-Cl | H | H | $CH_3$ | $CH_3$ |
| Ex 26 (b) | - | 3-Cl | 4-Cl | H | $CH_2CH_3$ | $CH_2CH_3$ |
| Ex 27 (b) | - | 4-Cl | H | OH | $CH_3$ | $CH_3$ |
| Ex 28 (a) | CRL 41 355 | H | H | H | H | H |

Notes

(a) : dichlorhydrate

(b) : diméthanesulfonate

(c) : base libre

Les produits de formule I que l'on préfère eu égard à leurs propriétés neuropsychopharmacologiques sont :

- dans le tableau $I_a$
- la 1-méthyl-5-phényl-1,4,5,6-tétrahydropyrimidine et ses sels d'addition d'acide tels que le chlorhydrate,

et surtout
- la 5-(2-fluorophényl)-1,4,5,6-tétrahydropyrimidine et ses sels d'addition d'acide tels que le chlorhydrate,
- la 1-acétyl-5-phényl-1,4,5,6-tétrahydropyrimidine et ses sels d'addition d'acide tels que le chlorhydrate, et
- la 5-hydroxy-5-phényl-1,4,5,6-tétrahydropyrimidine et ses sels d'addition d'acide tels que le chlorhydrate;

- dans le tableau $I_b$
- la 2-méthyl-5-phényl-1,4,5,6-tétrahydropyrimidine et ses sels d'addition d'acide tels que le chlorhydrate; et

- dans le tableau $I_c$
- la 2,2-diméthyl-5-(2-fluorophényl)-1,2,3,4,5,6-hexahydropyrimidine et ses sels d'addition d'acide tels que le dichlorhydrate,

et surtout

- la 2,2-diméthyl-5-phényl-1,2,3,4,5,6-hexahydropyrimidine, et ses sels d'addition d'acide tels que le dichlorhydrate.

Les composés selon l'invention peuvent être préparés selon une méthode connue en soi par application de mécanismes réactionnels classiques.
Le procédé que l'on préconise ici consiste

(a) à faire réagir une 2-phényl-1,3-propanediamine de formule

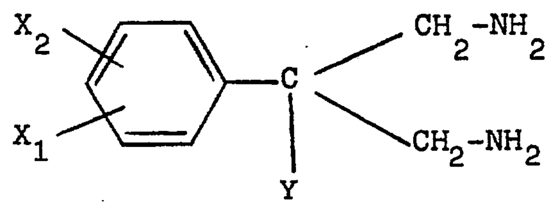

(II)

où Y, $X_1$ et $X_2$ sont définis comme indiqué ci-dessus, avec un réactif choisi par l'ensemble comprenant
(i) les formiates d'alkyle de formule
H-COO-Alk (IIIa)
où Alk représente un groupe alkyle inférieur en $C_1$-$C_3$ (de préférence $CH_2CH_3$), pour obtenir par cyclisation un composé de formule $I_a$ dans lequel $R_a$ est H ;
(ii) les alkylamidines de formule

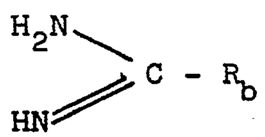

($III_b$)

où $R_b$ est défini comme indiqué ci-dessus, pour obtenir par cyclisation un composé de formule $I_b$ ; et
(iii) les aldéhydes et cétones de formule
$R_c^1$ et $R_c^2$ CO ($III_c$)
où $R_c^1$ et $R_c^2$ sont définis comme indiqué ci-dessus, pour obtenir par cyclisation un composé de formule $I_c$ ; et,

(b) si nécessaire, à faire réagir ledit dérivé de fromule $I_a$ où $R_a$ est H avec un réactif choisi parmi les agents d'alkylation et les agents d'acylation, pour obtenir un composé de formule $I_a$ dans lequel $R_a$ est un groupe alkyle en $C_1$-$C_4$ ou respectivement un groupe alcanoyle en $C_2$-$C_5$.

On a donné ci-après des informations utiles en ce qui concerne les modalités opératoires de ce procédé pour la synthèse des composés de formules $I_a$, $I_b$ et $I_c$.

<u>Synthèse des composés de formule $I_a$</u>

La réaction du stade (a) est mise en oeuvre pendant au moins 2 h, à raison d'au moins une mole de $III_a$ pour une mole de II. De façon avantageuse, la réaction de II avec $III_a$ est réalisée pendant 2,5-3,5 h à 100°C sous pression atmosphérique, d'une part, puis pendant 1 à 5 h, à une température de 100 à 160°C sous pression réduite, d'autre part.
L'alkylation du stade (b) est réalisée selon un mode connu en soi. De façon avantageuse on préconise ici d'utiliser comme agent d'alkylation un iodure d'alkyle en $C_1$-$C_4$ pour obtenir les dérivés de formule $I_a$ où $R_a$ est un groupe alkyle en $C_1$-$C_4$. De façon encore plus avantageuse, pour préparer les composés $R_a$=$CH_3$, $CH_2CH_3$ ou $CH(CH_3)_2$ on recommande de faire réagir un composé de formule $I_a$ où $R_a$ est H avec un mélange de formaldéhyde et d'acide carboxylique de formule
HZ–COOH (IV)
où Z est H, $CH_3$ ou respectivement $CH_2CH_3$, pour obtenir le dérivé N-alkyle correspondant où $R_a$ est $CH_3$, $CH_2CH_3$ ou respectivement $CH(CH_3)_2$.
La réaction avec le mélange HCHO + IV (dans lequel ledit mélange intervient comme solvant et réactif) est mise en oeuvre à 45-55°C jusqu'à la fin du dégagement de $CO_2$ puis à reflux pendant au moins 1 h. Le cas échéant, elle peut parfois impliquer, quand Y est OH, le blocage préalable de la fonction hydroxyle au moyen d'un groupe protecteur approprié, puis, l'élimination de ce groupe protecteur après que la N-alkylation ait été réalisée.
La réaction de N-acylation du stade (b) peut être réalisée selon un mode connu en soi. On peut, par exemple faire réagir un composé de formule $I_a$ dans lequel $R_a$ est H avec un réactif de N-acylation notamment choisi parmi les halogénures et anhydrides d'acide de formules

$Z_1$-CO-Hal (V) et $(Z_1$-CO$)_2$O ( VI)
où $Z_1$ est un groupe alkyle en $C_1$-$C_4$ et Hal est un atome d'halogène, notamment F, Cl ou Br (l'halogène préféré étant ici Cl).

Cette réaction de N-acylation est avantageusement mise en oeuvre en présence d'un accepteur de protons (notamment la pyridine ou la picoline qui peuvent également intervenir en tant que solvants) à raison d'au moins 2 moles d'halogénure V ou d'au moins 1 mole d'anhydride VI, pour 1 mole de composé $I_a$ où $R_a$ est H. Elle peut être réalisée à la température ambiante (15-20°C) ou à une température plus élevée, pendant au mois 2 h.

Le cas échéant, on peut avoir intérêt à protéger la fonction hydroxyle quand Y est OH pour effectuer la N-acylation comme indiqué ci-dessus pour la N-alkylation.

Synthèse des composés de formule $I_b$

On fait réagir au reflux 1 mole de 2-phényl-1,3-propanediamine II avec au moins 1 mole d'alkylamidine $III_b$ (de préférence 1,5 à 2,5 moles de $III_b$ pour 1 mole de II), dans un solvant approprié (notamment un alcanol inférieur en $C_1$-$C_3$, de préférence $C_2H_5OH$), jusqu'à ce que le dégagement de $NH_3$ soit terminé.

Synthèse des composés de formule $I_c$

On fait réagir à la température ambiante (15-20°C) 1 mole de 2-phényl-1,3-propanediamine II avec au moins 1 mole de composé carbonyl $III_c$, dans un solvant approprié (de préférence $H_2O$), pendant au moins 3 h (de préférence pendant 3 à 10 h), sous pression atmosphérique.

Les composés de formule I selon l'invention présentant la propriété commune d'agir sur le SNC. Ils présentent en particulier dans leur profil neuropsycho pharmacologique des effets antidépresseurs et sédatifs qui sont plus ou moins intenses. Plus précisément (i) les composés de formule $I_c$ agissent principalement en tant que sédatifs et possèdent des effets antidépresseurs peu marqués ; en revanche, (ii) les composés de formules $I_a$ et $I_b$ présentent principalement des effets antidépresseurs du SNC et, de façon accessoire, des effets sédatifs se manifestant aux doses élevées. Les composés de formule $I_a$ présentent également des propriétés stimumantes $\alpha$ -adrénergiques périphériques ainsi qu'on le verra plus loin. Les composés de formules $I_a$ et $I_b$ sont particulièrement intéressants en thérapeutique en tant que moyens antidépresseurs.

Selon l'invention on préconise une composition thérapeutique qui est caractérisée en ce qu'elle renferme, en association avec un excipient physiologiquement acceptable, au moins un dérivé choisi parmi les composés de formule I et leurs sels d'addition non-toxiques.

Bien entendu, dans une telle composition le principe actif, à savoir le composé de formule I ou l'un de ses sels non-toxiques, intervient en quantité pharmaceutiquement efficace.

Selon l'invention, on préconise l'utilisation d'une substance choisie parmi l'ensemble comprenant (i) les 5-phényl-1,4,5,6-tétrahydropyrimidines de formules $I_a$ et $I_b$, et (ii) leurs sels d'addition non-toxiques, pour l'obtention d'un médicament antidépresseur du SNC destiné à une utilisation en thérapeutique humaine vis-à-vis des dépressions et des états dépressifs.

Selon l'invention on préconise également l'utilisation d'une substance choisie parmi (i) les 5-phényl-1,2,3,4,5,6-hexahydropyrimidines de formule $I_c$ et (ii) leurs sels d'addition non toxiques, pour l'obtention d'un médicament sédatif, destiné à une utilisation en thérapeutique humaine vis-à-vis des états de surexcitation et nervosismes.

D'autres avantages et caractéristiques de l'invention seront mieux compris à la lecture qui va suivre d'exemples de préparation et de résultats d'essais pharmacologiques, l'ensemble de ces éléments n'étant nullement limitatif mais donné à titre d'illustration.

PREPARATION I

Obtention du chlorhydrate de 1-acétyl-5-phényl-1,4,5,6-tétrahydropyrimidine

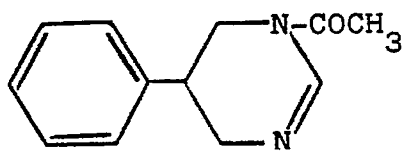

, HCl

(Exemple 7 ; No. de Code : CRL 41 378)

Dans de l'eau on dissout 40 g (0,186 mole) de chlorhydrate de 5-phényl-1,4,5,6-tétrahydropyrimidine hémihydraté, ajoute NaOH jusqu'à pH 11 et recueille la base libre formée (5-phényl-1,4,5,6-tétrahydropyrimidine). Après lavage à l'eau puis séchage à l'étuve à 60°C, on dissout 22,2 g (0,139 mole) de ladite base libre dans 555 ml de pyridine et ajoute 14,15 g (0,139 mole) d'anhydride acétique. On laisse en contact à température ambiante (15-20°C) pendant 20 h, évapore à sec, reprend le résidu d'évaporation au moyen

de 100 ml d'éthanol anhydre, acidifie au moyen d'éthanol contenant HCl et filtre les cristaux formés. Par recristallisation de l'éthanol anhydre on obtient 11 g (rendement : 33 %) de CRL 41 378. $F_{inst}$ = 200°C.

PREPARATION II

Obtention du chlorhydrate de 5-hydroxy-5-phényl-1,4,5,6-tétrahydropyrimidine.

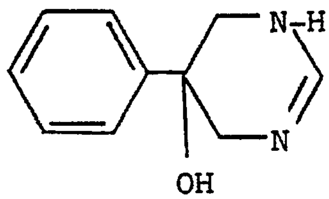

, HCl

(Exemple 8 ; No. de Code : CRL 41 382)

On porte à 100°C pendant 3 h un mélange constitué de 5,56 g (0,0335 mole) de dichlorhydrate de 1,3-diamino-2-phényl-2-propanol ($F_{inst}$ = 242°C) et de 2,479 g (0,0335 mole ; 2,7 ml) de formiate d'éthyle. On chauffe ensuite à 100°C sous pression réduite (vide obtenu au moyen d'une trompe à eau)pendant 1 h. On refroidit, reprend le milieu réactionnel avec $CH_3OH$, acidifie avec de l'éthanol chlorhydrique, évapore à sec, triture le résidu d'évaporation par de l'éther, de l'acétone et du chloroforme. On filtre pour recueillir le précipité formé, puis par recristallisation du mélange éthanoldiéthyléther,(1:1) v/v on obtient 6 g (rendement : 84 %) de CRL 41 382. $F_{inst.}$ = 206°C.

Analyse { % $Cl^-$ mesuré : 17,04 %
% $Cl^-$ théorique : 16,70 %

PREPARATION III

Obtention de chlorhydrate de 5-(2-fluorophényl)-1,4,5,6-tétrahydropyrimidine

Exemple 6 ; No. de Code : CRL 41 329).

On chauffe à 100°C pendant 3 h sous pression atmosphérique un milieu réactionnel obtenu par addition de 7,96 ml de formiate d'éthyle à 20 g (0,119 mole) de 2-(2-fluorophényl)-1,3-propanediamine, puis à 150°C pendant 1,50 h sous pression réduite (vide obtenu au moyen d'une trompe à eau). On refroidit, traite avec $CH_3OH$, acidifie au moyen d'éthanol chlorhydrique, évapore à sec, triture le résidu d'évaporation par de l'éther, de l'acétone puis du chloroforme. On filtre les cristaux formés, et par recristallisation dans le mélange $CH_3COCH_3$-$CH_3CH_2OH$ (1:1) v/v on obtient 10 g (rendement : 39 %) de CRL 41 329. $F_{inst.}$ = 210°C

Analyse { % $Cl^-$ mesuré : 16,50%
% $Cl^-$ théorique : 16,55%

PREPARATION IV

Obtention du dichlorhydrate de 2,2-diméthyl-5-(2-fluorophényl)-1,2,3,4,5,6-hexahydropyrimidine.

(Exemple 22 ; No. de Code : CRL 41 330).

On coule 194,6 g (1,22 mole ; 62,4 ml) de brome dans une solution de 227,8 g de NaOH dans 1700 ml d'eau à -3°C. On ajoute 122 g de 2-(2-fluorophényl)-1,3-propanedicarboxamide ($F_{inst.}$ = 206-208°C) en 5 fois, agite à 0°C jusqu'à l'obtention d'une solution claire, à température ambiante (15-20°C) pendant 1 heure puis à 70°C pendant 0,75 h. On refroidit et extrait par 6 fois 200 ml de chloroforme.

On ajoute dans la phase chloroformique ainsi obtenue [qui contient la 2-(2-fluorophényl)-1,3-propanediamine] et sans laver ladite phase avec de l'eau, 50 ml d'acétone et du sulfate de magnésium, puis on agite à la température ambiante (15-20°C) pendant une nuit. On filtre, évapore à sec le filtrat, reprend le résidu d'évaporation avec de l'éther de pétrole, puis filtre les cristaux formés. On reprend les cristaux par de l'éther de pétrole à reflux, filtre un insoluble à chaud, place le filtrat au congélateur puis recueille le précipité qui se forme et qu'on filtre et sèche sous vide. On obtient ainsi 38 g (rendement : 41 %) de 2,2-diméthyl-5-(2-fluorophényl)-1,2,3,4,5,6-hexahydropyrimidine.

| | | |
|---|---|---|
| Analyse | % N mesuré : | 13,50 % |
| | % N théorique : | 13,46 % |

A partir de la base libre ainsi obtenue on forme le dichlorhydrate. Dans ce but on acidifie au moyen d'éthanol chlorhydrique la base libre mise en solution dans $CH_3OH$. On évapore la solution résultante et sèche le résidu d'évaporation à 50°C sous vide. On obtient ainsi 51 g (rendement : 99,3 %) de CRL 41 330.

PREPARATION V

Obtention de la 2,2-diméthyl-5-hydroxy-5-phényl-1,2,3,4,5,6-hexahydropyrimidine

(Exemple 24 : No. de Code : CRL 41 365)

a) 1,3-Dichloro-3-phényl-2-propanol

Dans une solution de 100 g (0,787 mole) de 1,3-dichloroacétone dans 1000 ml d'éther anhydre, sous atmosphère d'azote et à - 60°C, on coule en 1,50 h , 289 ml (0,866 mole) d'une solution de bromure de phenylmagnesium 3M dans l'éther. Après 10 minutes on ajoute dans le milieu réactionnel 83,5 g d'acide acétique en solution dans 126 ml d'éther. On laisse la température monter à + 4°C et on coule 315 ml d'eau. On décante la phase éthérée qu'on lave à l'eau et sèche sur $MgSO_4$. On filtre, évapore à sec et distille le résidu d'évaporation sous vide, pour obtenir 82,3 g (rendement : 51 %) de 1,3-dichloro-2-phényl-2-propanol.
$Eb_{0,5\ mmHg}$ = 102°C (0,5 mmHg correspond à 66,6 Pa).

b) 1,3-Diphtalimido-2-phényl-2-propanol

On chauffe au bain-marie à 100°C un mélange de 30,6 g (0,149 mole) de 1,3-dichloro-2-phényl-2-propanol, 55,5 g (0,300 mole) de phtalimide de potassium et 300 ml de diméthylformamide, pendant 4 h. On refroidit, évapore jusqu'à ce que le volume du milieu réactionnel résultant ait diminué de moitié, filtre l'insoluble formé qu'on lave avec $CH_3OH$ puis sèche pour obtenir 40 g (rendement : 63 %) de 1,3-diphtalimido-2-phényl-2-propanol.

c) Dichlorhydrate de 2-hydroxy-2-phényl-1,3-propanediamine

On dissout 40 g (0,094 mole) de 1,3-diphtalimido-2-phényl-2-propanol dans 400 ml du mélange $C_2H_5OH$-$H_2O$ (95:5) v/v. On ajoute 10,2 g d'hydrate d'hydrazine et porte le milieu réactionnel au reflux pendant 2 h. Après refroidissement et acidification avec HCl 12N, on filtre l'insoluble et évapore à sec. On reprend le résidu d'évaporation avec 50 ml $H_2O$ et 100 ml $CHCl_3$ puis écarte par filtration l'insoluble formé. On extrait la phase aqueuse par 8 x 50 ml de $CHCl_3$,rassemble les phases organiques, sèche sur $MgSO_4$, filtre (pour écarter $MgSO_4$) puis évapore à sec. On reprend le résidu d'évaporation avec de l'éther et filtre le précipité formé que l'on recueille. Par recristallisation de l'éthanol on obtient 8 g (rendement : 35 %) de dichlorhydrate de 2-hydroxy-2-phényl-1,3-propanediamine. $F_{inst}$ = 242°C.

| | | |
|---|---|---|
| Analyse | % $Cl^-$ mesuré : | 29,65 % |
| | % $Cl^-$ théorique: | 29,62 % |

d) CRL 41 365

On dissout 4,4 g (0,01841 mole) du dichlorhydrate obtenu au stade c) ci-dessus, dans 50 ml d'eau. On ajoute 2,2 g de soude en pastilles et 3 ml d'acétone. On laisse une nuit au repos à la température ambiante (15-20°C), extrait par 3 x 100 ml de $CHCl_3$, lave la phase chloroformique avec 30 ml d'eau, sèche sur $MgSO_4$ puis filtre pour écarter $MgSO_4$. On évapore à sec le fitrat, par recristallisation de l'éther de pétrole (fraction bouillant à 45-60°C) on obtient 2,6 g (rendement 68,5 %) de CRL 41 365. F = 108°C.

PREPARATION VI

Obtention du dichlorhydrate de 5-phényl-1,2,3,4,5,6-hexahydropyrimidine

(Exemple 28 ; No de Code CRL 41 355)

On dissout 37,5 g (0,168 mole) de dichlorhydrate de 2-phényl-1,3-propanediamine dans 100 ml d'eau et ajoute 13,52 g (0336 mole) de soude en pastilles.On refroidit par un bain eau + glace et ajoute 17 ml d'une solution aqueuse de formaldéhyde à 30 % p/v. On laisse une nuit à la température ambiante (15-20°C), extrait par 5 x 50 ml de $CHCl_3$, lave la phase chloroformique avec 25 ml d'eau, sèche sur $MgSO_4$ et filtre. On évapore le filtrat à sec, reprend le résidu d'évaporation par 500 ml de $C_2H_5OH$ anhydre, acidifie avec de l'éthanol chlorhydrique, recueille par filtration le précipité formé qu'on lave avec un peu d'éthanol anhydre et sèche sous vide. On obtient 24 g (rendement : 58 %) de CRL 41 365. $F_{inst}$ = 192°C

Analyse { % $Cl^-$ mesuré : 29,71 %
% $Cl^-$ théorique: 30,47 %

PREPARATION VII

Obtention du chlorhydrate de 2-méthyl-5-phényl-1,4,5,6-tétrahydropyrimidine

(Exemple 16 ; No. de Code : CRL 41 352)

On porte au reflux jusqu'à la fin du dégagement de $NH_3$ un mélange constitué de 20 g (0,0897 mole) de dichlorhydrate de 2-phényl-1,3-propanediamine, 10,76 g (0,269 mole) de soude en pastilles et 8,48 g (0,0897 mole) de chlorhydrate d'acétamidine. On refroidit le milieu réactionnel, filtre l'insoluble que l'on écarte, acidifie le filtrat au moyen d'éthanol chlorhydrique, évapore pour diminuer le volume de moitié et filtre l'insoluble formé. On dilue le filtrat avec un égal volume d'éther, filtre l'insoluble formé et évapore à sec. Par recristallisation du résidu d'évaporation du mélange acétoneéthanol (1:1) v/v on obtient 6 g (rendement : 32 %) de CRL 41 352. $F_{inst}$ = 220°C.

PREPARATION VIII

Obtention du chlorhydrate de 1-méthyl-5-phényl-1,4,5,6-tétrahydropyrimidine.

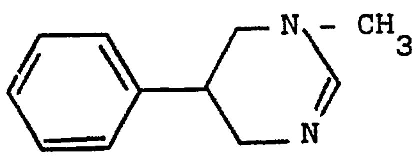

, HCl

(Exemple 2; No. de Code : CRL 41 337)

On dissout dans de l'eau 21 g (0,102 mole) de chlorhydrate de 5-phényl-1,4,5,6-tétrahydropyrimidine hémihydrate, ajoute NaOH jusqu'à pH 11 et filtre l'insoluble qu'on lave à l'eau puis recueille.

A la base libre ainsi obtenue (à savoir la 5-phényl-1,4,5,6-tétrahydropyrimidine) on ajoute 12 ml de formaldéhyde en solution aqueuse à 30% (p/v) et 10,6 ml d'acide formique à 99% (p/v). On chauffe à 45-55°C jusqu'à la fin du dégagement de $CO_2$, puis à reflux pendant 4 h. On évapore à sec, reprend le résidu d'évaporation avec de l'eau, alcalinise avec NaOH jusqu'à pH 11, extrait à l'éther. On lave la phase éthérée avec de l'eau, sèche sur $MgSO_4$, filtre (pour écarter $MgSO_4$) et ajoute de l'éthanol chlorhydrique. On décante la phase éthérée résultante, place le résidu huileux dans un dessicateur sous vide, en présence de KOH et $P_2O_5$, pendant 24 h. Le chlorhydrate attendu cristallise. On recueille 8 g (rendement : 37%) de CRL 41 337.

PREPARATION IX

Obtention du chlorhydrate de 5-(4-chlorophényl)-1,4,5,6-tétrahydropyrimidine.

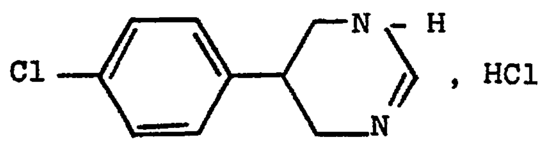

Exemple 1; No. de Code : CRL 41 336)

On dissout 30 g (0,1165 mole) de dichlorhydrate de 2-(4-chlorophényl)-1,3-propanediamine dans le minimum d'eau, ajoute NaOH jusqu'à pH 11, extrait par 4 x 100 ml de chloroforme, lave la phase chloroformique avec 50 ml d'eau, sèche sur $MgSO_4$, filtre et évapore à sec le filtrat pour obtenir 20,8 g (0,1127 mole) de 2-(4-chlorophényl)-1,3-propanediamine.

On ajoute à la base libre ainsi obtenue 8,34 g (0,1127 mole; 9,1 ml) de formiate d'éthyle. On chauffe le milieu réactionnel à 90-100°C pendant 3 h sous pression atmosphérique, puis à 150-155°C sous pression réduite (trompe à eau) pendant 1,50 h. On refroidit, ajoute du méthanol, acidifie avec de l'éthanol chlorhydrique et évapore à sec. On reprend le résidu d'évaporation avec $CH_3COCH_3$, filtre les cristaux qui se forment. Par recristallisation de ces cristaux au moyen du mélange $CH_3COCH_3$-$C_2H_5OH$ (1:1) v/v on obtient 10 g (rendement : 38%) de CRL 41 336. $F_{inst}$= 226-230°C.

On a résumé ci-après les résultats d'essais toxicologiques et neuropsychopharmacologiques qui ont été mis en oeuvre avec les produits selon l'invention. Dans ces essais, sauf indication contraire, lesdits produits ont été administrés par voie intrapéritonéale en solution dans de l'eau distillée sous un volume de 20 ml/kg chez la souris mâle, et sous un volume de 5 ml/kg chez le rat mâle.

A. ESSAIS RELATIFS AU CRL 41 378 (Ex. 7)

Les essais ont été réalisés en solution dans l'eau à pH 4,0-5,5. Le pH de la solution aqueuse de CRL 41378 varie en fonction de la concentration : il est de 4,0 à la concentration de 50 g/l, de 4,5 à 12,5 g/l, de 5,0 à 0,8 g/l et de 5,5 pour des concentrations de CRL 41 378 inférieures ou égales à 0,2 g/l.

I. TOXICITE

Chez la souris mâle (lots de trois animaux par dose), par voie I.P., la DL-O (dose maximale non mortelle) du CRL 41 378 est supérieure à 256 mg/kg, dose à laquelle on observe chez tous les animaux traités une dyspnée, une salivation importante et des convulsions 10 minutes après administration.

II. COMPORTEMENT GLOBAL ET REACTIVITES

Des lots de 3 animaux sont observés avant, puis 0,25 h, 0,50 h, 1 h, 2 h, 3 h et 24 h après l'administration de CRL 41 378. On constate

1°) chez la souris

à la dose de 1 mg/kg :

- aucune modification nette du comportement et des réactivités par rapport aux animaux témoins ;

à la dose de 4 mg/kg :

- une mydriase modérée pendant 3 h, l'intensité maximale étant atteinte 1 h après administration ;

à la dose de 16 mg/kg :

- une salivation modérée 1 h après administration,
- une hypothermie modérée,
- une mydriase modérée 1 h après administration ; et,

à la dose de 64 mg/kg :

- une sédation pendant 3 h,
- une diminution de la fréquence respiratoire se manifestant, après administration, entre T + 0,5 h et T +1 h,

- une hypothermie modérée,
- une augmentation de la réactivité au toucher et une vasodilatation des pattes pendant 0,5 h,
- une salivation se manifestant après administration, entre T + 1 h et T + 2 h, et
- une mydriase pendant 3 h ;

2°) chez le rat

à la dose de 0,5 mg/kg :

- une mydriase modérée pendant 2 h, l'intensité maximale étant atteinte 0,5 h après administration ;

à la dose de 2 mg/kg :

- une mydriase pendant 3 h, l'intensité maximale étant également atteinte 0,5 h après administration ;

à la dose de 32 mg/kg :

- une diminution de la fréquence respiratoire et du tonus musculaire pendant 1 h,
- une exophtalmie pendant 1 h,
- une piloérection pendant 2 h,
- une salivation se manifestant 0,5 h après administration,
- une vasodilatation des pattes et du museau pendant 0,5 h, et
- une mydriase pendant 2 h, l'intensité maximale étant atteinte 0,5 h après administration.

III. INTERACTION AVEC L'APOMORPHINE

1°) Chez la souris

Le CRL 41 378 est administré à des lots de 6 souris 30 minutes avant l'injection sous-cutanée de 1 ou 16 mg/kg d'apomorphine. On observe que le CRL 41 378 antagonise l'hypothermie induite par l'apomorphine dès la dose de 0,5 mg/kg, et qu'il ne modifie pratiquement pas les comportements de verticalisation et les stéréotypies dûs à l'apomorphine.

2° Chez le rat

Le CRL 41 378 est administré à des lots de 12 rats 0,5 h avant l'injection sous-cutanée de 0,5 mg/kg d'apomorphine. On observe que, aux deux plus fortes doses utilisées (8 et 32 mg/kg), le CRL 41 378 semble diminuer modérément les stéréotypies induites par l'apomorphine.

IV. INTERACTION AVEC L'AMPHETAMINE

L'amphétamine (2 mg/kg) est injectée par voie intrapéritonéale à des lots de 6 rats, 30 minutes après l'administration de CRL 41 378. On constate qu'à la plus forte dose utilisée (32 mg/kg), le CRL 41 378 potentialise les stéréotypies amphétaminiques.

V. INTERACTION AVEC LA RESERPINE

Quatre heures après l'injection intrapéritonéale de 2,5 mg/kg de réserpine, des lots de 12 souris reçoivent le CRL 41 378. On note que le CRL 41 378, aux doses de 0,5 mg/kg à 4 mg/kg, antagonise l'hypothermie induite par la réserpine, et que cet effet tend à diminuer pour s'annuler à la dose de 16 mg/kg, la plus forte dose utilisée (64 mg/kg) étant inactive vis-à-vis de l'hypothermie réserpinique. On observe par ailleurs que, dès la dose de 0,5 mg/kg, le CRL 41 378 provoque une diminution modérée du ptôsis réserpinique 24 h après administration.

VI. INTERACTION AVEC L'OXOTREMORINE

Le CRL 41 378 est adminstré à des lots de 6 souris 0,5 h avant l'injection intrapéritonéale de 0,5 mg/kg d'oxotrémorine.

1°) Action sur la température

On constate que le CRL 41 378 dès la dose la plus faible (0,5 mg/kg) antagonise l'action hypothermisante de l'oxotrémorine. Cet antagonisme est très intense aux doses de 2 et 4 mg/kg, puis est moins important pour les plus fortes doses utilisées (16 et 64 mg/kg).

2°) Action sur les tremblements

On constate que le CRL 41 378 ne modifie pas l'intensité des tremblements induits par l'oxotrémorine.

3°) Action sur les symptômes cholinergiques périphériques

On observe que le CRL 41 378 ne modifie pratiquement pas les signes de stimulation cholinergique périphérique induits par l'oxotrémorine.

VII. ACTION SUR LE TEST DES QUATRE PLAQUES, LA TRACTION ET L'ELECTROCHOC

Le test est pratiqué sur des lots de 10 souris, 30 minutes après l'administration de CRL 41 378.
On constate que le CRL 41 378 ne modifie pas le nombre de passages punis, et qu'il ne provoque pas d'incoordination motrice. Aux deux plus fortes doses étudiées (16 et surtout 64 mg/kg) le CRL 41 378 aggrave fortement les effets létaux de l'électrochoc sans modifier les effets convulsivants.

VIII. ACTION SUR LA MOTILITE SPONTANEE

0,5 h après avoir reçu le CRL 41 378, les souris (6 par dose, 12 témoins) sont placées en actimètre où leur motilité est enregistrée pendant 30 minutes.
On observe que, à la dose de 16 mg/kg et surtout à la dose de 64 mg/kg, le CRL 41 378 diminue l'activité motrice spontanée de la souris.

IX. ACTION SUR L'AGRESSIVITE INTERGROUPES

Après avoir séjourné pendant 3 semaines dans chacune des moitiés d'une cage séparées par une cloison opaque, des groupes de 3 souris reçoivent le CRL 41 378. Une demi-heure plus tard, les deux groupes d'une même cage sont réunis par retrait de la cloison et on note le nombre de combats qui surviennent en 10 minutes. On constate que, dès la dose de 0,5 mg/kg mais surtout aux doses de 2 mg/kg, 4 mg/kg et 16 mg/kg, le CRL 41 378 diminue l'agressivite intergroupes.

X. ACTION VIS-A-VIS DE QUELQUES COMPORTEMENTS PERTURBES PAR DIVERS AGENTS

1°) Motilité réduite par habituation à l'enceinte

Après 18 heures de séjour dans les actimètres, les souris (6 par dose, 12 témoins) reçoivent le CRL 41 378. Elles sont aussitôt replacées dans leurs enceintes respectives et, une demi-heure plus tard, on enregistre leur motilité pendant 30 minutes. On constate que le CRL 41 378 n'entraîne pas une reprise de l'activité motrice de la souris habituée à son enceinte.

2°) Motilité réduite par agression hypoxique

0,5 h après avoir reçu le CRL 41 378, les souris (10 par dose, 20 témoins) sont soumises à une anoxie hypobare aiguë [dépression de 600 mmHg (i.e. environ 8 x $10^4$ Pa) en 90 secondes ; puis détente en 45 secondes] puis elles sont placées en actimètre où leur motilité est enregistrée pendant 10 minutes.
On observe que le CRL 41 378, aux doses de 1 mg/kg , 4 mg/kg et 16 mg/kg, n'entraîne pas de modification de la récupération motrice chez la souris dont la motilité a été déprimée à la suite d'un bref séjour dans une enceinte sous pression réduite. A la dose de 64 mg/kg, le CRL 41 378 aggrave les effets de l'anoxie hypobare et provoque la mortalité de tous les animaux traités.

3°) Anoxie asphyxique

Des lots de 10 souris reçoivent le CRL 41 378, 30 minutes avant l'administration intrapéritonéale de 32 mg/kg de triiodoéthylate de gallamine (agent curarisant de référence). On observe que le CRL 41 378, à la dose la plus forte utilisée (64 mg/kg) diminue modérément le délai d'apparition des convulsions et de la mort consécutives à l'anoxie asphyxique provoquée par un curarisant.

XI. INTERACTION AVEC LE BARBITAL

Une demi-heure après l'adminstration de CRL 41 378 des lots de 10 souris reçoivent une injection intrapéritonéale de barbital (220 mg/kg). On constate que le CRL 41 378 ne modifie pratiquement pas la durée du sommeil barbiturique.

XII. ACTION SUR LE "DESESPOIR COMPORTEMENTAL"

Une demi-heure après avoir reçu le CRL 41 378, des lots de 6 souris sont placés dans un bécher rempli d'eau sur une hauteur de 6 cm. On note la durée totale d'immobilité entre la 2ème et la 6ème minutes suivant l'immersion. On observe que le CRL 41 378 diminue modérément la durée d'immobilité de la souris placée en immersion forcée.

XIII. CONCLUSIONS

Les résultats des essais entrepris mettent en évidence que le CRL 41 378 présente dans son profil neuropsychopharmacologique :
- des effets antidépresseurs se manifestant dès les plus faibles doses et illustrés par un antagonisme des hypothermies induites par l'apomorphine, la réserpine et l'oxotrémorine chez la souris (cet antagonisme étant toutefois moins important voire nul pour les plus fortes doses utilisées), d'une part, et par un antagonisme modéré du "désespoir comportemental"chez la souris, d'autre part ;
- des effets sédatifs se manifestant aux doses élevées et objectivés par
- une sédation chez la souris,
- une diminution importante de l'activité motrice spontanée de la souris,
- une diminution de l'agressivité intergroupes chez la souris (à des doses qui ne diminuent pas l'activité motrice spontanée),
- un antagonisme modéré des mouvements stéréotypés induits par l'apomorphine chez le rat ; et
- des effets de stimulation $\alpha$-adrénergique périphérique, qui se manifestent aux doses élevées, notamment par
- une composante de stimulation $\alpha_1$ objectivée par une salivation et une mydriase (chez la souris et le rat) et une piloérection(chez le rat),
- une hyperthermie modérée chez la souris, et
- une vasodilatation périphérique (queue, pattes et museau) chez la souris et le rat.

Par ailleurs on constate que le CRL 41 378 aggrave les effets létaux des différents agents convulsivants étudiés (électrochoc, hypoxie hypobare, anoxie asphyxique).

En bref le CRL 41 378 s'est révélé comme étant un agent antidépresseur présentant à fortes doses des effets sédatifs. Il se rapproche des stimulants $\beta$-adrénergiques et se différencie (i) des antidépresseurs imipraminiques, par l'absence d'activité anticholinergique périphérique et l'absence d'antagonisme important de l'immobilité de désespoir,(ii) des antidépresseurs stimulants tels que la nomifensine et l'amineptine voire encore l'amphétamine, par l'absence d'effets stimulants du SNC, et (iii) des inhibiteurs de la monoamineoxydase, par l'absence d'antagonisme net du ptôsis réserpinique, d'une part, et la rapidité d'apparition de ses effets, d'autre part.

B. ESSAIS RELATIFS AU CRL 41 382 (Ex 8)

En procédant selon les protocoles opératoires donnés ci-dessus les résultats suivants ont été obtenus avec le CRL 41 382 en solution dans l'eau à pH6.

I. TOXICITE

Chez la souris mâle par voie I.P., la DL-0 du CRL 41 382 est supérieure à 256 mg/kg, et la DL-60 est de l'ordre de 500 mg/kg.

II. COMPORTEMENT GLOBAL ET REACTIVITES

On constate

1°) chez la souris

aux doses de 1 mg/kg, 4 mg/kg et 16 mg/kg :

- aucune modification nette du comportement et des réactivités par rapport aux animaux témoins ne recevant que de l'eau distillée ;

à la dose de 64 mg/kg :

- une sédation pendant 1 h,
- une diminution de la réactivité au toucher et de la réaction de peur pendant 0,5 h ; et

2°) chez le rat

aux doses de 0,5 mg/kg, 2 mg/kg et 8 mg/kg:

- un comportement, des réactivités, une variation du diamètre pupillaire et de la température rectale analogues à ceux du lot témoin ;

à la dose de 32 mg/kg :

- une mydriase modérée pendant 1 h.

III. INTERACTION AVEC L'APOMORPHINE

Chez la souris, le CRL 41 382 s'oppose à l'hypothermie induite par l'apomorphine dès la dose de 1 mg/kg et surtout aux doses de 16 à 64 mg/kg, mais il ne modifie pratiquement pas les comportements de verticalisation et les stéréotypies.

Chez le rat, le CRL 41 382 ne modifie pratiquement pas les stéréotypies induites par l'apomorphine.

IV. INTERACTION AVEC L'AMPHETAMINE

Le CRL 41 382 ne modifie pratiquement pas chez la souris les stéréotypies induites par l'amphétamine.

V. INTERACTION AVEC LA RESERPINE.

Aux doses de 4 mg/kg et 16 mg/kg, le CRL 41 382 s'oppose à l'hypothermie induite par la réserpine. A la plus forte dose utilisée (64 mg/kg), le CRL 41 382 n'a aucun effet sur l'hypothermie réserpinique.

On constate par ailleurs que le CRL 41 382 ne modifie pratiquement pas le ptôsis réserpinique.

VI. INTERACTION AVEC L'OXOTREMORINE

Le CRL 41 382 s'oppose à l'hypothermie induite par l'oxotrémorine dès la dose de 1 mg/kg ; l'antagonisme est très intense à la dose de 64 mg/kg.

Le CRL 41 382 ne modifie pratiquement pas les tremblements ni les signes de stimulation cholinergique périphérique dûs à l'oxotrémorine.

VII. ACTION SUR LE TEST DES QUATRE PLAQUES, LA TRACTION ET L'ELECTROCHOC.

Le CRL 41 382 ne modifie pratiquement pas le nombre de passages punis. Il ne provoque pas d'incapacité motrice majeure. Il ne s'oppose pas aux effets convulsivants mais aggrave (à la dose de 64 mg/kg) les effets létaux de l'électrochoc.

VIII. ACTION SUR LA MOTILITE SPONTANEE

Le CRL 41 382 ne modifie pas l'activité motrice spontanée de la souris.

IX. ACTION SUR L'AGRESSIVITE INTERGROUPES

On observe que le CRL 41 382 ne modifie pratiquement pas le nombre de combats chez la souris.

X. ACTION VIS-A-VIS DE QUELQUES COMPORTEMENTS PERTURBES PAR DIVERS AGENTS

1°) Motilité réduite par habituation à l'enceinte

Le CRL 41 382 n'entraîne pas de reprise de l'activité motrice chez la souris habituée à son enceinte.

2° ) Motilité réduite par agression hypoxique

Aux doses de 16 mg/kg et 64 mg/kg, le CRL 41 382 entraîne une augmentation de la récupération motrice chez la souris dont la motilité a été diminuée à la suite d'un séjour bref dans une enceinte à pression réduite.

3°) Anoxie asphyxique

Le CRL 41 382 ne modifie pratiquement pas le délai d'apparition des convulsions et de la mort consécutives à une anoxie provoquée par un curarisant tel que le triiodoéthylate de gallamine.

XI. INTERACTION AVEC LE BARBITAL

On constate que le CRL 41 382 ne modifie prati quement pas la durée du sommeil barbiturique.

XII. ACTION SUR LE "DESESPOIR COMPORTEMENTAL"

On observe que le CRL 41 382 ne modifie pas la durée d'immobilité de la souris placée en immersion forcée.

XIII. CONCLUSIONS

Les résultats des essais résumés ci-dessus mettent en évidence que le CRL 41 382 présente dans son profil neuropsychopharmacologique
- des effets antidépresseurs se manifestant dès les plus faibles doses utilisées et illustrés par un antagonisme des hypothermies induites par l'apomorphine, l'oxotrémorine et la réserpine chez la souris (néanmoins l'antagonisme de l'hypothermie réserpinique n'apparaît plus à la forte dose utilisée pour le CRL 41 382) ; et
- des effets sédatifs se manifestant de façon fugace aux plus fortes doses utilisées (sans que l'on ait décelé une diminution de l'activité motrice).

Le fait bénéfique que le CRL 41 382 entraîne une augmentation modérée de la récupération motrice chez la souris après hypoxie hypobare aiguë, en l'absence de toute stimulation motrice même frustre et de toute action anticonvulsivante, est actuellement inexpliqué.

En bref le CRL 41 382 est un agent antidépresseur très actif ayant aux doses élevées une composante sédative de faible intensité.

Le profil général de l'activité du CRL 41 382 chez l'animal rapproche cette substance des stimulants β-adrénergiques. En effet, l'absence d'activité anticholinergique périphérique et l'absence d'antagonisme de l'immobilité de "désespoir" différencient le CRL 41 382 des antidépresseurs imipraminiques. L'absence d'effets stimulants différencie le CRL 41 382 des antidépresseurs stimulants comme la nomifensine, l'amineptine ou même l'amphétamine. Enfin, l'absence d'antagonisme du ptôsis réserpinique et la rapidité d'apparition des effets du CRL 41 382 permettent de la différencier des inhibiteurs de la monoamine-oxydase.

C. ESSAIS RELATIFS AU CRL 41 329 (Ex 6)

En procédant selon les protocoles opératoires donnés ci-dessus on a obtenu les résultats suivants pour le CRL 41 329 administré par voie I.P. en solution dans l'eau à pH 6.

a) Toxicité

Chez la souris mâle par voie I.P., la DL-0 (dose maximale non mortelle) du CRL 41 329 est supérieure à 64 mg/kg, dose à laquelle on observe chez tous les animaux traités une salivation, une sédaction et une vasodilatation de la queue, et la DL-100 (dose minimale mortelle pour la totalité des animaux) est de l'ordre de 128 mg/kg, dose à laquelle tous les animaux ont des convulsions asphyxiques 10 à 15 minutes après administration,la mort intervenant en 15 à 25 minutes.

b) Comportement global et réactivités

On observe

1°) chez la souris

- à la dose de 0,5 mg/kg :

- une mydriase modérée pendant 2 h ;

- à la dose de 2 mg/kg :

- une sédation (2 animaux sur 3) pendant 0,5 h, et
- une mydriase modérée et brève pendant 0,5 h ;

- à la dose de 8 mg/kg :

- une sédation (2 animaux sur 3) pendant 0,5 h, et
- une mydriase faible et brève pendant 0,5 h, et

- à la dose de 32 mg/kg :

- une sédation (3/3) 0,25 h après administration, puis (2/3) 0,5 h après administration ;

2°) chez le rat

- à la dose de 0,25 mg/kg :

- une mydriase modérée pendant 3 h ;

- à la dose de 1 mg/kg :

- une mydriase importante pendant 3 h ;

- à la dose de 4 mg/kg :

- une sédation (3/3) pendant 2 h,
- une diminution de la réactivité au toucher et du tonus musculaire,
- une piloérection (1/3) pendant 1 h, et
- une mydriase importante pendant au moins 3 h (la mydriase se manifestant encore 24 h après administration) ; et,

- à la dose de 16 mg/kg :

- une sédation (3/3) pendant 3 h,
- une diminution de la réactivité au toucher et du tonus musculaire, et
- une mydriase importante pendant 3 h (la mydriase se manifestant encore 24 h après administration).

c) Résultats des essais neuropsychopharmacologiques

On constate que dans son profil neuropsychopharmacologique le CRL 41 329 présente
- des effets antidépresseurs se manifestant à toutes les doses utilisées et illustrés par un antagonisme important des hypothermies induites par l'apomorphine, l'oxotrémorine et la réserpine (sans diminution de la durée d'immobilité dite de "désespoir") ;
- des effets sédatifs qui se manifestent aux doses élevées et sont objectivés par
- une sédation avec hypomotilité,
- une diminution de la récupération motrice après agression hypoxique,
- une diminution de l'agressivité intergroupes chez la souris,
- une hypothermie fugace de faible intensité.

On observe également que le CRL 41 329 présente en outre des effets modestes susceptibles de refléter une stimulation α -adrénergique périphérique, d'une part, et provoque chez la souris une aggravation des effets létaux de l'électrochoc et du triiodoéthylate de gallamine, d'autre part. De façon paradoxale il potentialise les stéréotypies amphétaminiques.

D. ESSAIS RELATIFS AU CRL 41 352 (Ex 16)

En procédant selon les protocoles opératoires donnés ci-dessus on a obtenu les résultats résumés ci-après pour le CRL 41 352 administré par voie I.P. en solution dans de l'eau à pH 5,5.

a) Toxicité

Chez la souris mâle par voie I.P., la DL-0 du CRL 41 352 est supérieure à 64 mg/kg, dose à laquelle on observe chez tous les animaux traités une sédation et une diminution de la fréquence respiratoire. La DL-30 est de l'ordre de 128 mg/kg.

b) Comportement global et réactivités

Aux doses de 0,5 mg/kg, 2 mg/kg, 8 mg/kg et 32 mg/kg on n'observe chez la souris aucune modification nette du comportement et des réactivités. En revanche chez le rat on observe une mydriase aux doses de 4 mg/kg et 16 mg/kg et une diminution de la fréquence respiratoire à la dose de 16 mg/kg.

c) Résultats des essais neuropsychopharmacologiques

On constate que le CRL 41 352 présente dans son profil neuropsychopharmacologique
- des effets antidépresseurs très nets, objectivés par un antagonisme important (dès les plus faibles doses utilisées) des hypothermies induites par l'apomorphine et l'oxotrémorine , d'une part, et un antagonisme plus modéré de l'hypothermie induite par la réserpine, d'autre part, mais sans diminution de la durée d'immobilité dite de "désespoir" ;

- un effet stimulant très modéré, se manifestant aux doses élevées et objectivé par une reprise de l'activité motrice chez la souris habituée à son enceinte ; et
- des effets sédatifs se manifestant aux doses très élevées et illustrés par une diminution du nombre des combats dans le test de l'agressivité intergroupes.

En bref, le CRL 41 352 s'est révélé comme étant principalement un agent antidépresseur ayant une très légère composante stimulante et une très faible composante sédative.

E. ESSAIS RELATIFS AU CRL 41 365 (Ex 24)

En procédant selon les protocoles opératoires donnés ci-dessus on a obtenu les résultats résumés ci-après pour le CRL 41 365 administré par voie I.P. en solution dans de l'eau à pH 5,5.

a) Toxicité

Chez la souris mâle par voie I.P. la DL-0 (dose maximale non mortelle) est supérieure à 512 mg/kg.

b) Comportement global et réactivités

On constate

1° ) chez la souris

- aux doses de 16 et 64 mg/kg :

- aucune modification nette du comportement et des réactivités par rapport aux animaux témoins ne recevant que de l'eau distillée ;

- à la dose de 256 mg/kg :

- une sédation pendant 1 h ;
- une diminution de la fréquence cardiaque pendant 1 h ;
- une diminution de la réactivité au toucher pendant 0,5 h ; et
- une hypothermie pendant 3 h qui atteint son intensité maximale (- 1,6°C) 0,5 h après administration ; et,

2°) chez le rat

- à la dose de 8 mg/kg :

- aucune modification nette du comportement et des réactivités par rapport aux animaux témoins ;

- à la dose de 32 mg/kg :

- une sédation et une diminution de la fréquence respiratoire pendant 1 h ; et
- une diminution de la réactivité au toucher pendant 0,5 h ; et,

- à la dose de 128 mg/kg :

- une sédation et une diminution de la fréquence respiratoire pendant 3 h ; et
- une diminution de la réactivité au toucher pendant 1 h.

c) Résultats des essais neuropsychopharmacologiques

Eu égard aux essais qui ont été réalisés selon les protocoles donnés ci-dessus, le CRL 41 365 s'est révélé comme étant une substance essentiellement sédative (sédation avec diminution des réactivités chez le rat et la souris, hypothermie chez la souris, diminution très modérée de la durée d'immobilité de la souris placée en immersion forcée).

Par ailleurs ce produit potentialise, à toutes les doses étudiées, les stéréotypies amphétaminiques (cette potentialisation serait probablement de nature pharmacocinétique).

F. ESSAIS RELATIFS AU CRL 41 336 (Ex. 1)

I. TOXICITE

Chez la souris mâle, par voie I.P., la DL-0 (dose maximale non mortelle) du CRL 41 336 est supérieure à 64 mg/kg, dose à laquelle on observe chez tous les animaux traités une sédation et une diminution de la fréquence respiratoire, et la DL-100 (dose minimale mortelle pour la totalité des animaux) est de l'ordre de environ 200-210 mg/kg, la mort intervenant 10 à 13 minutes après administration.

La DL-60 I.P. chez la souris mâle est de l'ordre de environ 128 mg/kg. A cette dose les animaux présentent une sédation et une diminution de la fréquence respiratoire, la mort pour 60% des animaux intervenant 24 h après administration.

II. COMPORTEMENT GLOBAL ET REACTIVITES

Des lots de 3 animaux sont observés avant, puis 0,25 h, 0,50 h, 1 h, 2 h, 3 h et 24 h après l'administration de CRL 41 329. On constate

1°) chez la souris

aux doses de 0,5 à 8 mg/kg :

- aucune modification nette du comportement et des réactivités;

à la dose de 32 mg/kg :

- une sédation pendant 0,25 h,
- une diminution de la fréquence respiratoire de 1 à 3 heures,
- une hypothermie modérée (-1,3°C) de 1 à 3 heures,
- une vasodilatation des oreilles et de la queue de 0,5 h à 3 h ;

2°) chez le rat

aux doses de 0,25 et 1 mg/kg :

- un comportement, des réactivités, des variations de la température rectale et du diamètre pupillaire sensiblement comparables à ceux des animaux du lot témoin ;

à la dose de 4 mg/kg :

- une mydriase pendant 2 heures (maximale 0,5 h après administration) ; et,

à la dose de 16 mg/kg :

- une sédation pendant 3 h,
- une diminution de la fréquence respiratoire pendant 3 h,
- une mydriase pendant 3 h (ayant son intensité maximale 0,5 h après administration),
- une piloérection pendant 1 h, et
- une vasodilatation (au niveau des pattes) pendant 2 h.

III. ETUDE NEUROPSYCHOPHARMACOLOGIQUE

Les résultats des essais entrepris mettent en évidence que le CRL 41 336 présente dans son profil neuropsychopharmacologique:
- des effets antidépresseurs illustrés par un antagonisme net des hypothermies induites par l'apomorphine et l'oxotrémorine, d'une part, et plus modéré de l'hypothermie induite par la réserpine, d'autre part ;
- des effets sédatifs, objectivés par
- une sédation chez la souris et le rat,
- une diminution de l'activité motrice spontanée,
- une diminution de la motilité après hypoxie hypobare aigüe [dépression de 600 mmHg (i.e. environ 8 x $10^4$Pa) en 90 secondes; puis détente en 45 secondes] chez la souris,
- une diminution de l'agressivité intergroupes chez la souris, et
- une hypothermie (fugace) chez la souris ; et
- des effets de stimulation$\alpha$-adrénergique périphérique, qui se manifestent aux doses élevées, notamment par une myriase et une piloérection chez le rat.

On observe que, à fortes doses, le CRL 41 336 (i) provoque une aggravation de l'effet létal de l'électrochoc, et (ii) diminue chez le rat et la souris les stéréotypies induites par l'apomorphine. La diminution des stéréotypies induites par l'apomorphine suggère que le CRL 41 336 peut avoir un mode d'action de type antidopaminergique postsynaptique.

G. ESSAIS RELATIFS AU CRL 41 337 (Ex 2)

I. TOXICITE

Chez la souris mâle par voie I.P. la DL-0 du CRL 41 337 est supérieure à 256 mg/kg, la DL-60 est de l'ordre de environ 500 mg/kg et la DL-100 est inférieure ou égale à environ 1000 mg/kg.

II. COMPORTEMENT GLOBAL ET REACTIVITES

Selon les modalités opératoires données ci-dessus on constate

1°) chez la souris

aux doses de 2 à 32 mg/kg :

- aucune modification nette du comportement et des réactivités par rapport aux témoins; et,

à la dose de 128 mg/kg :

- une hypothermie modérée (-1,6°C, une heure après administration) durant pendant 3 h ;

2° ) chez le rat

aux doses de 1 et 4 mg/kg :

- aucune modification nette du comportement et des réactivités par rapport aux témoins; et,

aux doses de 16 et 64 mg/kg :

- une mydriase.

III. ETUDE NEUROPSYCHOPHARMACOLOGIQUE

Les résultats des essais entrepris mettent en évidence que le CRL 41 337 présente dans son profil neuropsychopharmacologique :
- des effets antidépresseurs qui se manifestent par un antagonisme des hypothermies induites par l'apomorphine, la réserpine et l'oxotrémorine, sans modification de la durée d'immobilité dite de désespoir ;
- des effets sédatifs se traduisant par
- une diminution de l'activité motrice,
- une diminution de l'agressivité intergroupes, et
- une hypothermie ;
- des effets de stimulation α-adrénergique périphérique se manifestant par
- une mydriase chez le rat, et
- un antagonisme du ptôsis induit par la réserpine.

On observe par ailleurs que, à doses élevées, le CRL 41 337 entraîne paradoxalement une amélioration de la récupération motrice chez la souris après hypoxie hypobare aigüe, et une reprise de l'activité motrice chez la souris habituée à son enceinte. De plus le CRL 41 337 entraîne, à dose élevée une aggravation des effets létaux de l'électrochoc.

En bref, le CRL 41 337 se comporte, eu égard aux résultats des essais qui ont été entrepris, comme une substance agissant principalement en tant que moyen antidépresseur. Son profil neuropsychopharmacologique rappelle par certains aspects ceux des stimulants β-adrénergiques (antagonismes des hypothermies induites par l'apomorphine la réserpine et l'oxotrémorine, mais sans effet au niveau du test de l'immobilité dite de désespoir). Enfin le CRL 41 337 se différencie des substances imipraminiques par le fait qu'il n'a aucune action anticholinergique et pas d'effet sur l'immobilité de désespoir, d'une part, et des antidépresseurs, tels que la nomifensine, par le fait qu'il ne présente aucune stimulation de l'activité motrice spontanée, d'autre part.

H. ESSAIS COMPLEMENTAIRES

Des essais complémentaires ont été entrepris avec les CRL 41 329 (Exemple 6), CRL 41 378 (Exemple 7), CRL 41 382 (Exemple 8) et CRL 41 352 (Exemple 16), qui sont les produits les plus intéressants selon

l'invention. Ces essais ont porté sur
- l'étude des propriétés neuropsychopharmacologiques par voie gastrique,
- l'étude des propriétés cardiovasculaires par voie intraduodénale et/ou intraveineuse, et
- l'étude de l'interaction avec le 5-hydroxytryptophane chez la souris pré-traité par un agent inhibiteur de la monoamine oxydase (IMAO).

1. Etude neuropsychopharmacologique par voie gastrique

Chaque produit à tester est adminstré par voie gastrique, sous un volume de 20 ml/kg, à des souris mâles 0,50 h avant l'injection sous-cutanée de 16 mg/kg d'apomorphine. Ce protocole a pour but de vérifier la présence ou l'absence d'effets antidépresseurs par voie gastrique par interaction avec l'apomorphine.

On observe que dès les doses de 0,25 mg/kg (CRL 41 329), 0,50 mg/kg (CRL 41 378 et CRL 41 352) et 2 mg/kg (CRL 41 382) les produits étudiés s'opposent à l'hypothermie induite par l'apomorphine. L'antagonisme de l'hypothermie induite par l'apomorphine augmente avec la dose de chaque produit à tester.

On constate que, par voie gastrique, un effet important de type antidépresseur se manifeste pour chacun des produits à tester, et que cet effet est similaire à celui observé après administration desdits produits par voie I.P.

2. Etude cardiovasculaire

Des lots de 2 chiens adultes (pesant chacun 10 à 18 kg), anesthésiés au nembutal, recoivent chaque produit à tester (i) par voie I.D. aux doses successives de 0,1 mg/kg, 0,5 mg/kg, 1 mg/kg, 2,5 mg/kg, 5 mg/kg, 10 mg/kg et 20 mg/kg, puis (ii), le cas échéant, par voie I.V. à la dose de 10 mg/kg. On apprécie également les effets de 10 µg/kg d'isoprénaline et/ou 2 µg/kg de noradrénaline après administration de la dose cumulée de 39,1 mg/kg I.D.

On mesure la pression artérielle globale, la pression artérielle diastolique, la fréquence cardiaque, les débits artériels fémoraux, cérébraux et saphènes, les températures rectale et cutanée, et, la pression des gaz (essentiellement $O_2$) dissous dans le sang artériel.

On observe ce qui suit.

a) Le CRL 41 329 (Ex 6) par voie I.D. a tendance à augmenter la pression artérielle, à diminuer la fréquence cardiaque en entraînant des arythmies respiratoires, et diminue les débits artériels fémoraux et vertébraux chez le chien anesthésié; il entraîne une mydriase et une salivation importante et augmente les températures rectale et cutanée; il ne modifie pas de façon nette les effets de l'isoprénaline sur la pression artérielle diastolique et la fréquence cardiaque, en revanche, il diminue l'hypertension induite par la noradrénaline.

b) Le CRL 41 378 (Ex. 7) administré chez le chien anesthésié est sans effet sur la pression artérielle par voie I.D. et est hypotenseur par voie I.V.; il diminue les débits des artères fémorale et vertébrale; il augmente la température rectale mais ne modifie pas de façon nette la température cutanée; il diminue les effets de l'isoprénaline sur la fréquence cardiaque et diminue l'hypertension à la noradrénaline.

c) Le CRL 41 382 (Ex. 8) administré par voie I.D. chez le chien anesthésié est hypertenseur, mais pratiquement sans effet sur la fréquence cardiaque, il diminue les débits fémoraux et vertébraux ainsi que la température cutanée, en revanche il augmente la température rectale; par voie I.V., il est hypotenseur bradycardisant, et diminue les débits artériels fémoraux et vertébraux; il ne modifie pas les effets de l'isoprénaline et augmente l'hypertension provoquée par la noradrénaline; selon ces essais, le CRL 41 382 semble présenter un effet stimulant α-adrénergique.

3. Interaction avec le 5-hydroxytryptophane

Le protocole mis en oeuvre a pour but de rechercher l'activité de chaque produit selon l'invention vis-à-vis des tremblements induits par le 5-hydroxytryptophane (5-HTP) associé à un agent IMAO (ici le nialamide).

Chaque produit àtester, en solution dans de l'eau distillée, est administré par voie I.P. sous un volume de 20 ml/kg chez la souris mâle.

Des lots de 10 souris reçoivent le nialamide (20 mg/kg, par voie gastrique) à l'instant T - 18 h, chaque produit à tester à l'instant T - 0,5 h, puis, le (±)-5-HTP (20 mg/kg, par voie I.P.) à l'instant T. Immédiatement après l'injection du (±)-5-HTP, les souris sont placées individuellement dans des boîtes en plastique transparentes. Les tremblements généralisés, les tremblements partiels et les secousses de la tête ("head twitch") sont notés en tout ou rien pendant 2 h.

On observe que (i) dès la dose de 1 mg/kg, le CRL 41 378 potentialise nettement les tremblements partiels et moyennement les secousses de la tête, la potentialisation étant très intense à partir de la dose de 16 mg/kg; (ii) dès la dose de 2 mg/kg, les CRL 41 329 et CRL 41 352 potentialisent nettement les tremblements partiels et les secousses de la tête, la potentialisation étant très intense aux doses de 8 à 32 mg/kg

qui ont été étudiées; et (iii) le CRL 41 382 entraîne une potentialisation modérée des tremblements partiels et ne modifie pratiquement pas les secousses de la tête.

Selon ce protocole on constate que les produits à tester se différencient par leurs profils neuropsychopharmacologiques des antidépresseurs tricycliques (tels que la désipramine), des substances amphétaminiques (telles que l'amphétamine), et des moyens libérateurs de sérotonine (tels que la fenfluramine) qui potentialisent tous les effets du 5-HTP en présence d'un IMAO. Au contraire lesdits produits se rapprocheraient plutôt des stimulants b-adrénergiques, qui exercent des effets disparates vis-à-vis du 5-HTP, tels que l'isoprénaline (potentialisation nette des tremblements et des secousses de la tête), le fénotérol (potentialisation nette des tremblements et potentialisation moyenne des secousses de la tête), la ritodrine et, à un degré moindre, le salbutamol (potentialisation moyenne des tremblements sans modification des secousses de la tête) et la terbutaline et l'ociprénaline (pas de modification des tremblements et des secousses de la tête).

Plus précisément les CRL 41 329 et CRL 41 352 auraient suivant ledit protocole des effets analogues à ceux de l'isoprénaline, le CRL 41 378 se rapprocherait plutôt du fénotérol, et le CRL 41 382 se rapprocherait plutôt de la ritodrine ou du salbutamol.

Par ailleurs on a observé que les composés selon l'invention, notamment les CRL 41 329, CRL 41 378, CRL 41 382 et CRL 41 352, sont dépourvus d'effets tératogènes néfastes chez la lapine gravide.

Les essais cliniques qui ont été entrepris ont permis de déterminer que la dose quotidienne à administrer per os chez l'homme adulte, dans le traitement des dépressions et des états dépressifs, est en général comprise entre 3 et 120 mg pour les produits de formules $I_a$ et $I_b$ selon l'invention.

En particulier on a obtenu d'excellents résultats en thérapeutique humaine en administrant quodidiennement per os aux adultes (i) 2 à 3 comprimés ou gélules renfermant chacun 25 à 35 mg de CRL 41 378 ou de CRL 41 382, (ii) 2 à 3 comprimés ou gélules renfermant chacun 30 mg de CRL 41 329, et (iii) 2 comprimés renfermant chacun 1,5 à 3 mg de CRL 41 352. Chez les patients ambulatoires ayant une symptomatologie dépressive se manifestant par une asthénie, un ralentissement, une inhibition tant psycho-intellectuelle que motrice, le CRL 41 352 s'est révélé particulièrement efficace.

En ce qui concerne les produits de formule $I_c$ qui se sont révélés en clinique intéressants en tant que substances sédatives, la posologie recommandée pour les états de surexcitation et le nervosisme est de l'ordre de 300 à 2000 mg per os par jour chez l'homme adulte.

**Revendications pour les états contractants BE, CH, DE, FR, GB, IT, LI, LU, NL, SE**

1. Dérivé de 5-phényl-1,4,5,6-tétrahydropyrimidine, caractérisé en ce qu'il est choisi parmi l'ensemble comprenant

(i) les 5-phényl-hydropyrimidines de formule

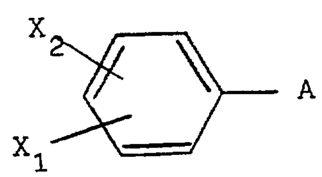

(I)

dans laquelle $X_1$ et $X_2$, identiques ou différents, représentent chacun H, F, Cl, Br ou $CF_3$; et A représente un groupe polyhydropyrimidinyle de structure

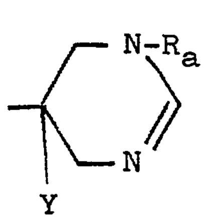 , 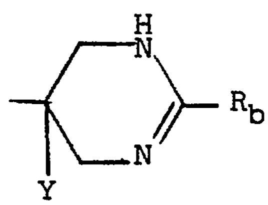 ou 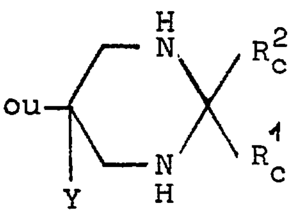

où Y représente H ou OH; $R_a$ représente un groupe alkyle en $C_1$–$C_4$ ou un groupe alcanoyle en $C_2$–$C_5$, $R_a$ pouvant représenter l'atome d'hydrogène quand au moins un des groupes $X_1$, $X_2$ et Y est différent de H; $R_b$ représente un groupe alkyle en $C_1$–$C_3$; $R_c^1$ et $R_c^2$ , identiques ou différents, représentent chacun l'atome d'hydrogène ou un groupe alkyle en $C_1$–$C_3$; et,

(ii), leurs sels d'addition.

2. Dérivé selon la revendication 1, caractérisé en ce qu'il est choisi parmi l'ensemble comprenant

(i) les 5-phényl-1,4,5,6-tétrahydropyrimidines substituées de formule

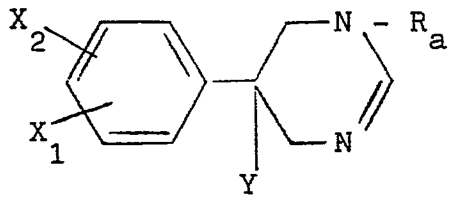

$(I_a)$

dans laquelle $X_1$ et $X_2$, identiques ou différents, représentent chacun H, F, Cl, Br ou $CF_3$; Y est H ou OH; et, $R_a$ est alkyle en $C_1$–$C_4$ ou alcanoyle en $C_2$–$C_5$, $R_a$ pouvant représenter l'atome d'hydrogène quand un au moins des groupes $X_1$, $X_2$ et Y est différent de H; et,
(ii), leurs sels d'addition.
3. Dérivé selon la revendication 1, caractérisé en ce qu'il est choisi parmi l'ensemble comprenant
(i) les 2-alkyl-5-phényl-1,4,5,6-tétrahydropyrimidines répondant à la formule

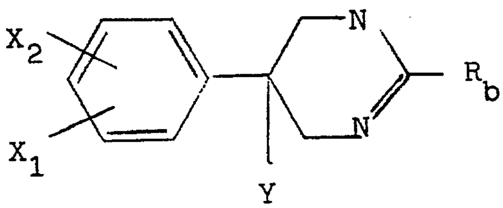

$(I_{b'})$

dans laquelle $X_1$ et $X_2$, identiques ou différents, représentent chacun H, F, Cl, Br ou $CF_3$; Y est H ou OH; et $R_b$ est un groupe alkyle en $C_1$–$C_3$; et,
(ii) leurs sels d'addition.
4. Dérivé selon la revendication 1, caractérisé en ce qu'il est choisi parmi l'ensemble comprenant
(i), les 5-phényl-1,2,3,4,5,6-hexahydropyrimidines répondant à la formule

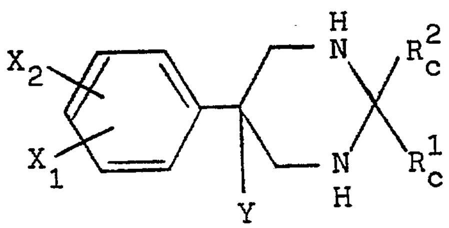

$(I_c)$

dans laquelle $X_1$ et $X_2$, identiques ou différents, représentent chacun H, F, Cl, Br ou $CF_3$; Y est H ou OH; et, $R_c^1$ et $R_c^2$ identiques ou différents, représentent chacun l'atome d'hydrogène ou un groupe alkyle en $C_1$-$C_3$; et,
(ii) leurs sels d'addition.
5. Dérivé selon la revendication 1, caractérisé en ce qu'il est choisi parmi l'ensemble comprenant
a) les 5-phényl-1,4,5,6-tétrahyropyrimidines substituées de formule

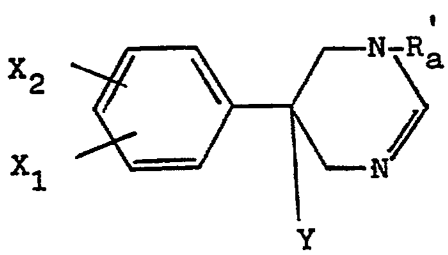

$(I'_a)$

dans laquelle $X_1$ et $X_2$, identiques ou différents, représentent chacun H, F, Cl, Br ou $CF_3$; Y est H ou OH; et, $R'_a$ est H, alkyle en $C_1$-$C_4$ ou alcanoyle en $C_2$-$C_5$, $R'_a$ étant différent de H si, simultanément on a (i) Y = H, (ii) $X_2$ = H et (iii) $X_1$ = H ou 4-Cl;
b) les 2-alkyl-5-phényl-1,4,5,6-tétrahydropyrimidines répondant à la formule

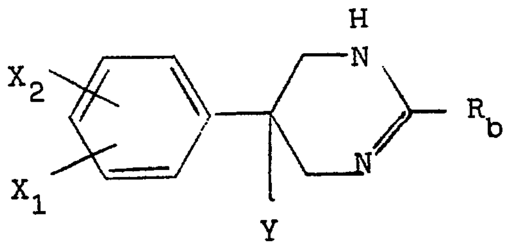

($I_{b'}$)

dans laquelle
$X_1$, $X_2$ et Y sont définis comme ci-dessus; et
$R_b$ est un groupe alkyle en $C_1$-$C_3$;
c) les 5-phényl-1,2,3,4,5,6-hexahydropyrimidines répondant à la formule

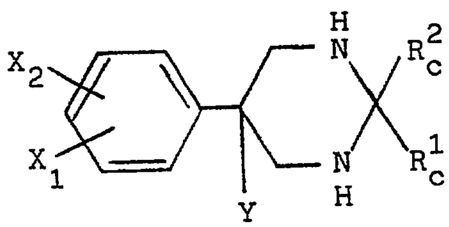

($I_c$)

dans laquelle $X_1$, $X_2$ et Y sont définis comme indiqués ci-dessus; et
$R_c^1$ et $R_c^2$, identiques ou différents, représentent chacun l'atome d'hydrogène ou un groupe alkyle en $C_1$-$C_3$; et,
d) leurs sels d'addition.

6. 5-(2-Fluorophényl)-1,4,5,6-tétrahydropyrimidine et ses sels d'addition.

7. 1-Acétyl-5-phényl-1,4,5,6-tétrahydropyrimidine et ses sels d'addition.

8. 5-Hydroxy-5-phényl-1,4,5,6-tétrahydropyrimidine et ses sels d'addition.

9. 2-Méthyl-5-phényl-1,4,5,6-tétrahydropyrimidine et ses sels d'addition.

10. 2,2-Diméthyl-5-hydroxy-5-phényl-1,2,3,4,5,6-hexahydropyrimidine et ses sels d'addition.

11. Composition thérapeutique, caractérisée en ce qu'elle renferme, en association avec un excipient physiologiquement acceptable, au moins un dérivé choisi parmi les composés de formule I et leurs sels d'addition non-toxiques selon la revendication 1.

12. Utilisation d'une substance choisie parmi l'ensemble comprenant les dérivés de 5-phényl-1,4,5,6-tétrahydropyrimidine de formules $I_a$ et respectivement $I_b$ selon la revendication 2 et respectivement la revendication 3, et leurs sels d'addition non-toxiques, pour l'obtention d'un médicament antidépresseur du SNC destiné à une utilisation en thérapeutique humaine vis-à-vis des dépressions et des états dépressifs.

13. Procédé de préparation d'un composé de formule I selon la revendication 1, caractérisé en ce qu'il comprend
a) la réaction d'une 2-phényl-1,3-propanediamine de formule

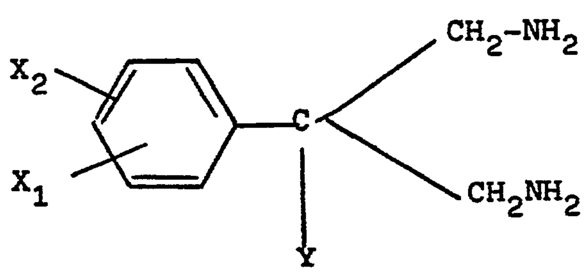

(II)

où Y, $X_1$ et $X_2$ sont définis comme indiqué ci-dessus, avec un réactif choisi parmi l'ensemble comprenant
(i) les formiates d'alkyle de formule
H-COO-Alk ($III_a$)
où AlK représente un groupe alkyle inférieur en $C_1$-$C_3$ (de préférence $CH_2CH_3$), pour obtenir par cyclisation un composé de formule

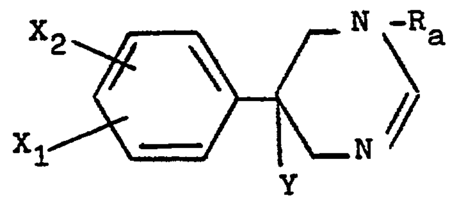

$(I_a)$

dans laquelle Y, $X_1$ et $X_2$ sont définis comme ci-dessus et $R_a$ est H;
(ii) les alkylamidines de formule

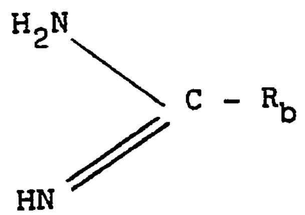

$(III_b)$

où $R_b$ est défini comme indiqué ci-dessus, pour obtenir par cyclisation un composé de formule

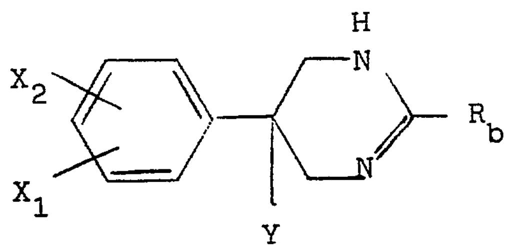

$(I_b)$

dans laquelle $X_1$, $X_2$, Y et $R_b$ sont définis comme ci-dessus; et
(iii) les aldéhydes et cétones de formule
$R_c^1$ et $R_c^2$ CO ($III_c$)
où $R_c^1$ et $R_c^2$ sont définis comme indiqué ci-dessus, pour obtenir par cyclisation un composé de formule

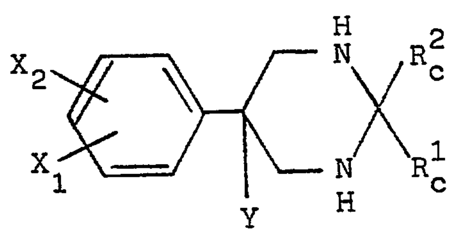

$(I_c)$

dans laquelle $X_1$, $X_2$, Y, $R_c^1$ et $R_c^2$ sont définis comme ci-dessus;
b) si nécessaire, à faire réagir ledit dérivé de formule $I_a$ où $R_a$ est H avec un réactif choisi parmi les agents d'alkylation et les agents d'acylation, pour obtenir un composé de formule $I_a$ dans lequel $R_a$ est un groupe alkyle en $C_1$-$C_4$ ou respectivement un groupe alcanoyle en $C_2$-$C_5$.

**Revendications pour les états contractants AT, ES, GR**

1. Procédé de préparation d'un dérivé de 5-phényl1,4,5,6-tétrahydropyrimidine choisi parmi l'ensemble comprenant
(i) les 5-phényl-hydropyrimidines de formule

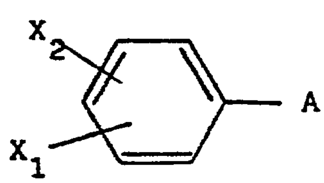

(I)

dans laquelle $X_1$ et $X_2$, identiques ou différents, représentent chacun H, F, Cl, Br ou $CF_3$; et A repré-

sente un groupe polyhydropyrimidinyle de structure

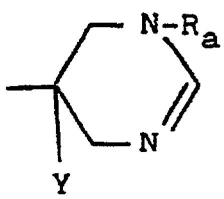

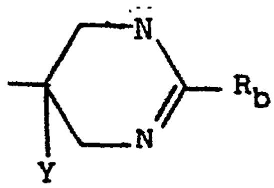

ou

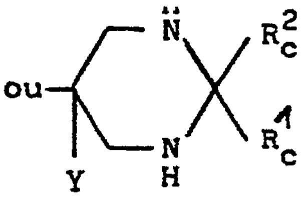

où Y représente H ou OH; $R_a$ représente un groupe alkyle en $C_1$–$C_4$ ou un groupe alcanoyle en $C_2$–$C_5$, $R_a$ pouvant représenter l'atome d'hydrogène quand au moins un des groupes $X_1$, $X_2$ et Y est différent de H; $R_b$ représente un groupe alkyle en $C_1$–$C_3$; $R_c^1$ et $R_c^2$ identiques ou différents, représentent chacun l'atome d'hydrogène ou un groupe alkyle en $C_1$–$C_3$; et,

(ii) leurs sels d'addition,

ledit procédé étant caractérisé en ce qu'il comprend

a) la réaction d'une 2-phényl-1,3-propanediamine de formule

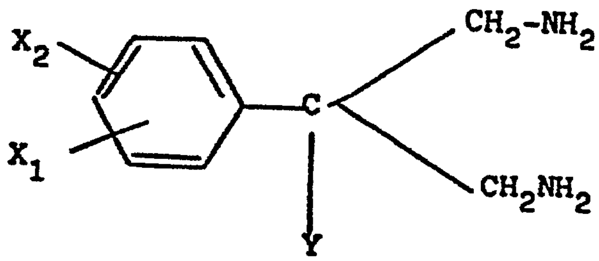

(II)

où Y, $X_1$ et $X_2$ sont définis comme indiqué ci-dessus, avec un réactif choisi parmi l'ensemble comprenant

(i) les formiates d'alkyle de formule H–COO–Alk ($III_a$) où Alk représente un groupe alkyle inférieur en $C_1$–$C_3$ (de préférence $CH_2CH_3$), pour obtenir par cyclisation un composé de formule

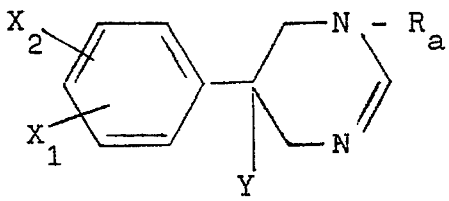

($I_a$)

dans laquelle Y, $X_1$ et $X_2$ sont définis comme ci-dessus et $R_a$ est H;

(ii) les alkylamidines de formule

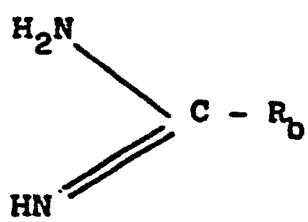

($III_b$)

où $R_b$ est défini comme indiqué ci-dessus, pour obtenir par cyclisation un composé de formule

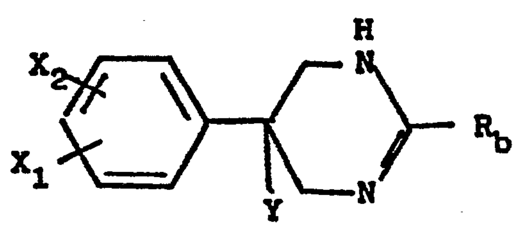

($I_b$)

dans laquelle $X_1$, $X_2$, Y et $R_b$ sont définis comme ci-dessus; et

(iii) les aldéhydes et cétones de formule $R_c^1$ et $R_c^2$ CO (IIIc)

où $R_c^1$ et $R_c^2$ sont définis comme indiqué ci-dessus, pour obtenir par cyclisation un composé de formule

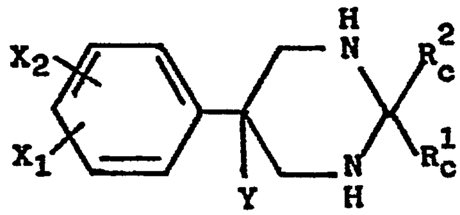

($I_c$)

dans laquelle $X_1$, $X_2$, Y, $R_c^1$ et $R_c^2$ sont définis comme ci-dessus;

b) si nécessaire, à faire réagir ledit dérivé de formule $I_a$ où $R_a$ est H avec un réactif choisi parmi les agents d'alkylation et les agents d'acylation, pour obtenir un composé de formule $I_a$ dans lequel $R_a$ est un groupe alkyle en $C_1$–$C_4$ ou respectivement un groupe alcanoyle en $C_2$–$C_5$.

2. Procédé suivant la revendication 1 pour la préparation d'un composé de formule

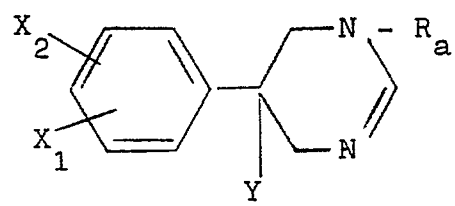

($I_a$)

dans laquelle $X_1$, $X_2$, Y et $R_a$ sont définis comme indiqué ci-dessus, et de ses sels d'addition, ledit procédé étant caractérisé en ce que l'on fait réagir pendant au moins 2 h, 1 mole de 2-phényl -1,3-propanediamine de formule II avec au moins 1 mole de formiate d'alkyle de formule $III_a$, à une température supérieure ou égale à 100°C, et en ce que, si nécessaire, on soumet le composé $I_a$ ainsi obtenu, où $R_a$ est H, à une réaction de N-alkylation ou de N-acylation pour obtenir un composé $I_a$ dans lequel $R_a$ est alkyle ou alcanoyle.

3. Procédé suivant la revendication 2, caractérisé en ce que l'on fait réagir au moins une mole de IIIa pour une mole de II, pendant 2,5 à 3,5 h à 100–160°C sous pression réduite.

4. Procédé suivant la revendication 1, pour la préparation d'un composé de formule

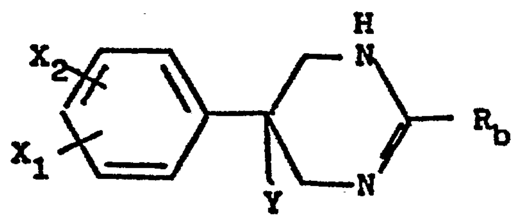

($I_b$)

dans laquelle $X_1$, $X_2$, Y et $R_b$ sont définis comme indiqué ci-dessus, et de ses sels d'addition, ledit procédé étant caractérisé en ce que l'on fait réagir 1 mole de 2-phényl1,3-propanediaminede formule II avec au moins 1 mole d'alkylamidine $III_b$, dans un solvant approprié, à la température de reflux du milieu réactionnel, jusqu'à ce que le dégagement de $NH_3$ soit terminé.

5. Procédé suivant la revendication 4, caractérisé en ce que l'on fait réagir 1 mole de II avec 1,5–2,5 moles de $III_b$.

6. Procédé suivant la revendication 1, pour la préparation d'un composé de formule

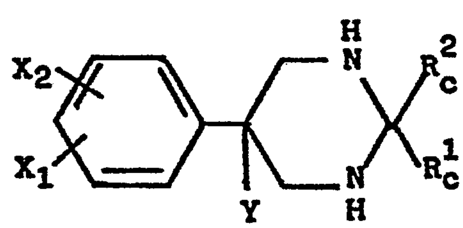

($I_c$)

dans laquelle $X_1$, $X_2$, Y, $R_c^1$ et $R_c^2$ sont définis comme ci-dessus, et de ses sels d'addition, ledit procédé

étant caractérisé en ce que l'on fait réagir à 15–25°C sous pression atmosphérique, 1 mole de 2-phényl-1,3-propanediamine de formule II avec au moins de composé carbonyle $III_c$, dans un solvant approprié, pendant au moins 3 h.

7. Procédé suivant l'une quelconque des revendications 1, 2 et 3, caractérisé en ce que $R_a$ est différent de H quand simultanément on a (i) Y = H, (ii) $X_2$ = H et (iii) $X_1$ = H ou 4–Cl.

8. Procédé suivant l'une quelconque des revendications 2 et 7, caractérisé en ce que $X_1$ = 2–F et $X_2$ = Y = $R_a$ = H, en ce que $R_a$ = $COCH_3$ et $X_1$ = $X_2$ = Y = H, et en ce que Y = OH et $X_1$ = $X_2$ = $R_a$ = H.

9. Procédé suivant l'une quelconque des revendications 4 et 5, caractérisé en ce que $R_b$ = $CH_3$ et $X_1$ = $X_2$ = Y = H.

10. Procédé suivant la revendication 6, caractérisé en ce que $X_1$ = 2–F, $X_2$ = Y = H et $R_c^1$ = $R_c^2$ = $CH_3$ et en ce que Y = OH, $X_1$ = $X_2$ = H et $R_c^1$ = $R_c^2$ = $CH_3$.

**Patentansprüche für die Vertragsstaaten BE, CH, DE, FR, GB, IT, LI, LU, NL, SE**

1. Neues 5-Phenyl-1,4,5,6-tetrahydropyrimidin-derivat, dadurch gekennzeichnet, daß es ausgewählt ist aus der Gruppe, bestehend aus:

(i) den 5-Phenyl-hydropyrimidinen der allgemeinen Formel:

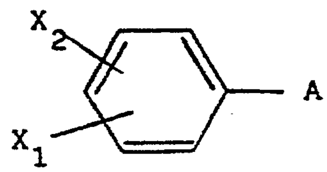

(I)

in welcher $X_1$ und $X_2$, die gleich oder voneinander verschieden sind, jeweils H, F, Cl, Br oder $CF_3$ bedeuten; und A eine Polyhydropyrimidinylgruppe der allgemeinen Struktur:

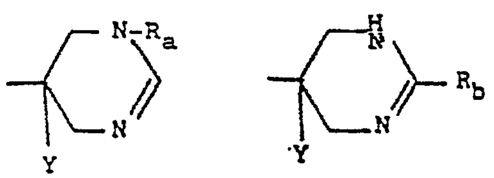

oder

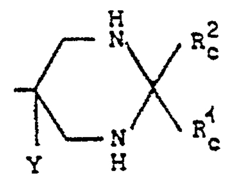

dargestellt, worin Y Wasserstoff oder OH bedeutet; $R_a$ eine $C_1$–$C_4$-Alkylgruppe oder eine $C_2$–$C_5$-Alkanoylgruppe bedeutet, und $R_a$ ferner Wasserstoff darstellen kann, wenn mindestens eine der Gruppen $X_1$, $X_2$ und Y von Wasserstoff verschieden ist; $R_b$ eine $C_1$–$C_3$-Alkylgruppe bedeutet; $R_c^1$ und $R_c^2$, die gleich oder voneinander verschieden sind, jeweils Wasserstoff oder eine $C_1$–$C_3$-Alkylgruppe darstellen; und

(ii) deren Additionssalze.

2. Derivat nach Anspruch 1, dadurch gekennzeichnet, daß es ausgewählt ist aus der Gruppe, bestehend aus

(i) den substituierten 5-Phenyl-1,4,5,6-tetrahydropyrimidinen der allgemeinen Formel:

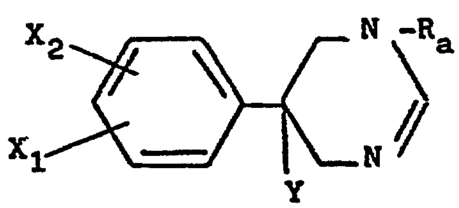

$(I_a)$

in welcher $X_1$ und $X_2$, die gleich oder voneinander verschieden sind, jeweils H, F, Cl, Br oder $CF_3$ bedeuten; Y Wasserstoff oder OH bedeutet; und $R_a$ eine $C_1$–$C_4$-Alkylgruppe oder eine $C_2$–$C_5$-Alkanoylgruppe bedeutet und $R_a$ ferner Wasserstoff darstellen kann, wenn mindestens eine der Gruppen $X_1$, $X_2$ und Y von Wasserstoff verschieden ist; und

(ii) deren Additionssalze.

3. Derivat nach Anspruch 1, dadurch gekennzeichnet, daß es ausgewählt ist aus der Gruppe, bestehend aus:

(i) den 2-Alkyl-5-phenyl-1,4,5,6-tetrahydropyrimidinen der allgemeinen Formel:

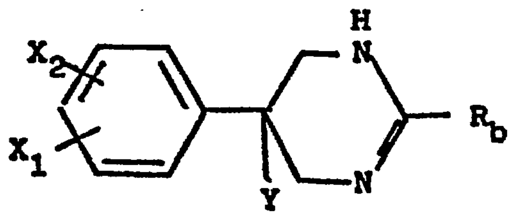

$(I_b)$

in welcher $X_1$ und $X_2$, die gleich oder voneinander verschieden sind, jeweils H, F, Cl, Br oder $CF_3$ bedeuten; Y Wasserstoff oder OH bedeutet; und $R_b$ eine $C_1$–$C_3$-Alkylgruppe bedeutet; und

(ii) deren Additionssalze.

4. Derivat nach Anspruch 1, dadurch gekennzeichnet, daß es ausgewählt ist aus der Gruppe, bestehend aus:

(i) den 5-Phenyl-1,2,3,4,5,6-hexahydropyrimidinen der allgemeinen Formel:

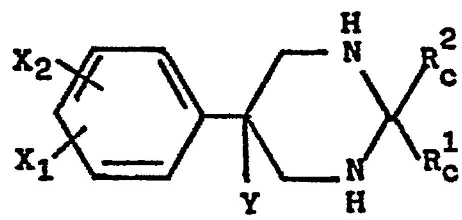

$(I_c)$

in welcher $X_1$ und $X_2$, die gleich oder voneinander verschieden sind, jeweils H, F, Cl, Br oder $CF_3$ bedeuten; Y Wasserstoff oder OH bedeutet; und $R_c^1$ und $R_c^2$, die gleich oder voneinander verschieden sind, jeweils Wasserstoff oder eine $C_1$–$C_3$-Alkylgruppe darstellen; und

(ii) deren Additionssalze.

5. Derivat nach Anspruch 1, dadurch gekennzeichnet, daß es ausgewählt ist aus der Gruppe, bestehend aus:

a) den substituierten 5-Phenyl-1,4,5,6-tetrahydropyrimidinen der allgemeinen Formel:

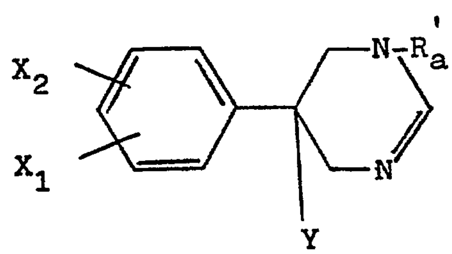

$(I'_a)$

in welcher $X_1$ und $X_2$, die gleich oder voneinander verschieden sind, jeweils H, F, Cl, Br oder $CF_3$ bedeuten; Y Wasserstoff oder OH bedeutet; und $R'_a$ Wasserstoff, eine $C_1$–$C_4$-Alkylgruppe oder eine $C_2$–$C_5$-Alkanoylgruppe darstellt, wobei $R'_a$ von Wasserstoff verschieden ist, wenn gleichzeitig (i) Y = H, (ii) $X_2$ = H und (iii) $X_1$ = H oder 4–Cl;

b) den 2-Alkyl-5-phenyl-1,4,5,6-tetrahydropyrimidinen der allgemeinen Formel:

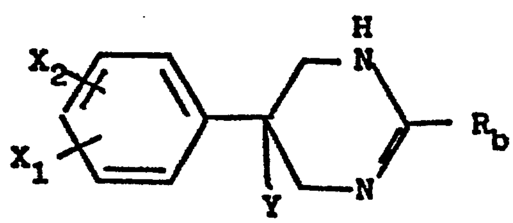

$(I_b)$

in welcher $X_1$, $X_2$ und Y die hier angegebene Bedeutung haben und $R_b$ eine $C_1$–$C_3$-Alkylgruppe darstellt;

c) den 5-Phenyl-1,2,3,4,5,6-hexahydropyrimidinen der allgemeinen Formel:

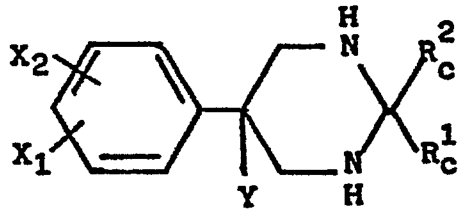

($I_c$)

in welcher $X_1$, $X_2$ und Y die hier angegebene Bedeutung haben; und $R_c^1$ und $R_c^2$, die gleich oder voneinander verschieden sind, jeweils Wasserstoff oder eine $C_1$–$C_3$-Alkylgruppe darstellen; und

d) deren Additionssalze.

6. 5-(2-Fluorphenyl)-1,4,5,6-tetrahydropyrimidin und dessen Additionssalze.

7. 1-Acetyl-5-phenyl-1,4,5,6-tetrahydropyrimidin und dessen Additionssalze.

8. 5-Hydroxy-5-phenyl-1,4,5,6-tetrahydropyrimidin und dessen Additionssalze.

9. 2-Methyl-5-phenyl-1,4,5,6-tetrahydropyrimidin und dessen Additionssalze.

10. 2,2-Dimethyl-5-hydroxy-5-phenyl-1,2,3,4,5,6-hexahydropyrimidin und dessen Additionssalze.

11. Therapeutisches Mittel, dadurch gekennzeichnet, daß es in Verbindung mit einem physiologisch zulässigen Bindemittel mindestens ein Derivat, ausgewählt aus den Verbindungen der allgemeinen Formel (I) und deren nicht-toxischen Additionssalzen, enthält.

12. Verwendung einer Substanz, ausgewählt aus der Gruppe, bestehend aus den 5-Phenyl-1,4,5,6-tetrahydropyrimidinderivaten der allgemeinen Formeln ($I_a$) bzw. ($I_b$), nach Anspruch 2, bzw. Anspruch 3, und deren nicht-toxischen Additionssalzen, zur Herstellung eines SNC-antidepressions-medikamentes, das zur Verwendung in der Humantherapie gegen Depressionen und depressive Zustände bestimmt ist.

13. Verfahren zur Herstellung einer Verbindung der allgemeinen Formel (I) gemäß Anspruch 1, dadurch gekennzeichnet, daß man

a) ein 2-Phenyl-1,3-propandiamin der allgemeinen Formel:

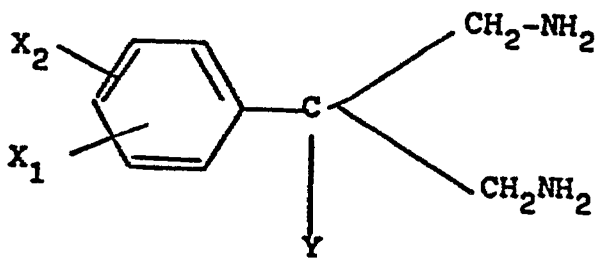

(II)

in welcher Y, $X_1$ und $X_2$ die oben angegebene Bedeutung haben, mit einem Reaktionsmittel, ausgewählt aus der Gruppe, bestehend aus

(i) den Alkylformiaten der allgemeinen Formel:

H–COO–Alk, ($III_a$) in welcher Alk eine $C_1$–$C_3$-Niederalkylgruppe (vorzugsweise $CH_2CH_3$) bedeutet, zur Herstellung einer Verbindung der allgemeinen Formel:

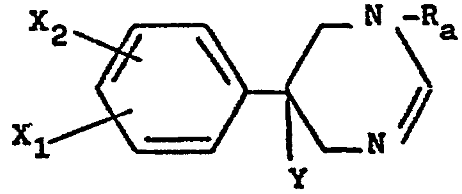

($I_a$)

in welcher Y, $X_1$ und $X_2$ die obige Bedeutung haben und $R_a$ für Wasserstoff steht; durch Cyclisierung;

(ii) den Alkylamidinen der allgemeinen Formel:

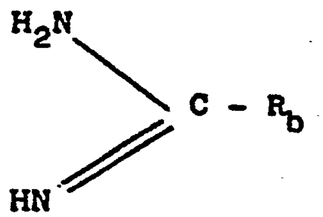

($III_b$)

in welcher $R_b$ die obige Bedeutung hat, zur Herstellung einer Verbindung der allgemeinen Formel:

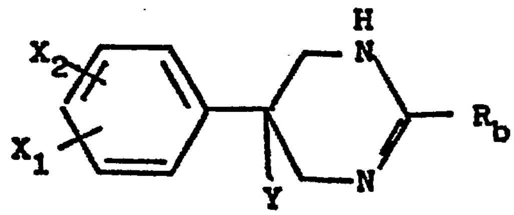 $(I_b)$

in welcher $X_1$, $X_2$, Y und $R_b$ die obige Bedeutung haben, durch Cyclisierung; und
(iii) den Aldehyden und Ketonen der allgemeinen Formel:

$$R_c^1\ R_c^2\ CO \qquad (III_c)$$

in welcher $R_c^1$ und $R_c^2$ die obige Bedeutung haben, zur Herstellung einer Verbindung der allgemeinen Fomel:

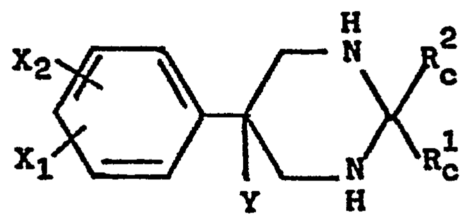 $(I_c)$

in welcher $X_1$, $X_2$, Y, $R_c^1$ und $R_c^2$ die obige Bedeutung haben; durch Cyclisierung umsetzt; und daß man
b) erforderlichenfalls dieses Derivat der allgemeinen Formel $(I_a)$, worin $R_a$ für Wasserstoff steht, mit einem Reaktionsmittel ausgewählt aus Alkylierungsmitteln und Acylierungsmitteln, zur Herstellung einer Verbindung der allgemeinen Formel $(I_a)$, in welcher $R_a$ eine $C_1$–$C_4$-Alkylgruppe, bzw. eine $C_2$–$C_5$-Alkanoylgruppe darstellt, umsetzt.

**Patentansprüche für die Vertragsstaaten AT, ES, GR**

1. Verfahren zur Herstellung eines 5-Phenyl-1,4,5,6-tetrahydropyrimidin-derivates, ausgewählt aus der Gruppe, bestehend aus:
(i) den 5-Phenyl-hydropyrimidinen der allgemeinen Formel:

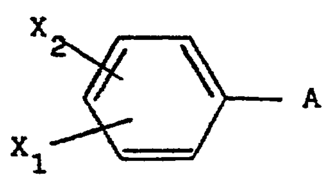 (I)

in welcher $X_1$ und $X_2$, die gleich oder voneinander verschieden sind, jeweils H, F, Cl, Br oder $CF_3$ bedeuten; und A eine Polyhydropyrimidinylgruppe der allgemeinen Struktur:

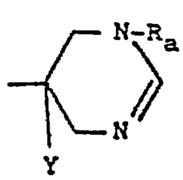 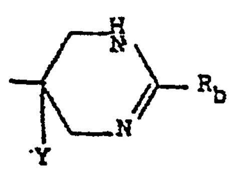 oder 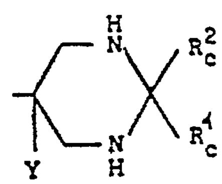

darstellt, worin Y Wasserstoff oder OH bedeutet; $R_a$ eine $C_1$–$C_4$-Alkylgruppe oder eine $C_2$–$C_5$-Alkanoylgruppe bedeutet, und $R_a$ ferner Wasserstoff darstellen kann, wenn mindestens eine der Gruppen $X_1$, $X_2$ und Y von Wasserstoff verschieden ist; $R_b$ eine $C_1$–$C_3$-Alkylgruppe bedeutet; $R_c^1$ und $R_c^2$, die gleich oder voneinander verschieden sind, jeweils Wasserstoff oder eine $C_1$–$C_3$-Alkylgruppe darstellen; und
(ii) von deren Additionssalzen,

dadurch gekennzeichnet, daß man

a) ein 2-Phenyl-1,3-propandiamin der allgemeinen Formel:

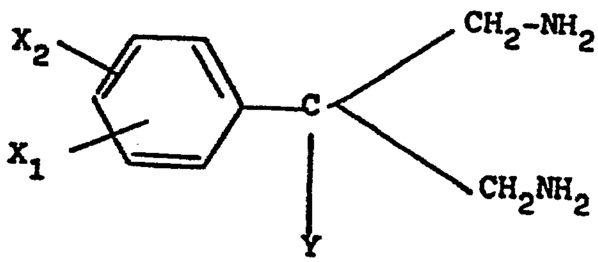

(II)

in welcher Y, $X_1$ und $X_2$ die obige Bedeutung haben,

mit einem Reaktionsmittel, ausgewählt aus der Gruppe, bestehend aus

(i) den Alkylformiaten der allgemeinen Formel:

H–COO–Alk, ($III_a$)

in welcher Alk eine $C_1$–$C_3$-Niederalkylgruppe (vorzugsweise $CH_2CH_3$) bedeutet, zur Herstellung einer Verbindung der allgemeinen Formel:

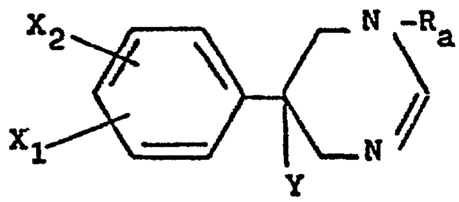

($I_a$)

in welcher Y, $X_1$ und $X_2$ die obige Bedeutung haben und $R_a$ für Wasserstoff steht, durch Cyclisierung;

(ii) den Alkylamidinen der allgemeinen Formel:

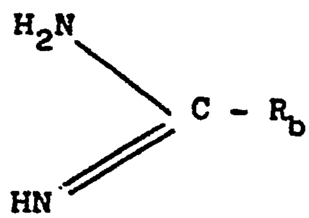

($III_b$)

in welcher $R_b$ die obige Bedeutung hat, zur Herstellung einer Verbindung der allgemeinen Formel:

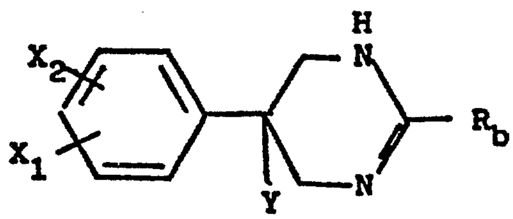

($I_b$)

in welcher $X_1$, $X_2$, Y und $R_b$ die obige Bedeutung haben, durch Cyclisierung; und

(iii) den Aldehyden und Ketonen der allgemeinen Formel:

$R_c^1$ und $R_c^2$ CO, ($III_c$)

in welcher $R_c^1$ und $R_c^2$, die obige Bedeutung haben, zur Herstellung einer Verbindung der allgemeinen Formel:

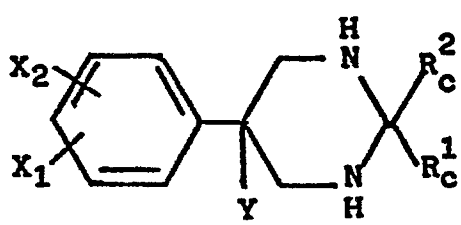

($I_c$)

in welcher $X_1$, $X_2$, Y, $R_c^1$ und $R_c^2$ die obige Bedeutung haben, durch Cyclisierung umsetzt; und daß man b) erforderlichenfalls dieses Derivat der allgemeinen Formel ($I_a$), worin $R_a$ für Wasserstoff steht, mit einem Reaktionsmittel, ausgewählt aus Alkylierungsmitteln und Acylierungsmitteln, zur Herstellung einer Verbindung der allgemeinen Formel ($I_a$), worin $R_a$ eine $C_1$–$C_4$-Alkylgruppe, bzw. eine $C_2$–$C_5$-Alkanoylgruppe ist, umsetzt.

2. Verfahren nach Anspruch 1 zur Herstellung einer Verbindung der allgemeinen Formel:

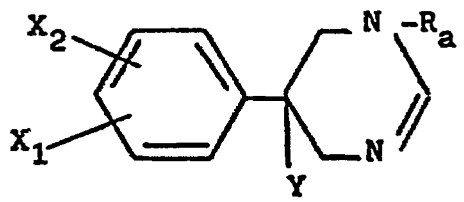

($I_a$)

in welcher $X_1$, $X_2$, Y und $R_a$ die obige Bedeutung haben, und von deren Additionssalzen, dadurch gekennzeichnet, daß man 1 Mol 2-Phenyl-1,3-propandiamin der allgemeinen Formel (II) während mindestens 2 Stunden mit mindestens 1 Mol Alkylformiat der allgemeinen Formel ($III_a$) bei einer Temperatur über oder gleich 100°C umsetzt und daß man erforderlichenfalls die so erhaltene Verbindung der allgemeinen Formel ($I_a$), worin $R_a$ für Wasserstoff steht, einer N-Alkylierungs- oder N-Acylierungsreaktion unterwirft unter Herstellung einer Verbindung der allgemeinen Formel ($I_a$) in welcher $R_a$ für Alkyl oder Alkanoyl steht.

3. Verfahren nach Anspruch 2, dadurch gekennzeichnet, daß man mindestens 1 Mol der Verbindung der allgemeinen Formel ($III_a$) pro 1 Mol der Verbindung der allgemeinen Formel (II) während 2,5 bis 3,5 Stunden bei 100°C unter Atmosphärendruck und sodann während 1 bis 5 Stunden bei 100–160°C unter vermindertem Druck umsetzt.

4. Verfahren nach Anspruch 1 zur Herstellung einer Verbindung der allgemeinen Formel:

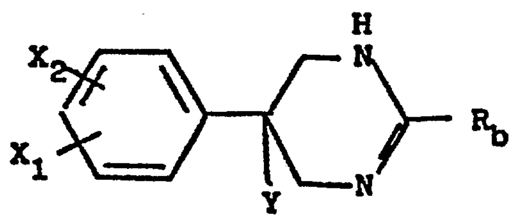

($I_b$)

in welcher $X_1$, $X_2$, Y und $R_b$ die obige Bedeutung haben, und von deren Additionssalzen, dadurch gekennzeichnet, daß man 1 Mol 2-Phenyl-1,3-propandiamin der allgemeinen Formel (II) mit mindestens 1 Mol Alkylamidin der allgemeinen Formel ($III_b$) in einem geeigneten Lösungsmittel bei der Rückflußtemperatur des Reaktionsmediums umsetzt, bis die $NH_3$-Entwicklung beendet ist.

5. Verfahren nach Anspruch 4, dadurch gekennzeichnet, daß man 1 Mol der Verbindung der allgemeinen Formel (II) mit 1,5 bis 2,5 Molen der Verbindung der allgemeinen Formel ($III_b$) umsetzt.

6. Verfahren nach Anspruch 1, zur Herstellung einer Verbindung der allgemeinen Formel:

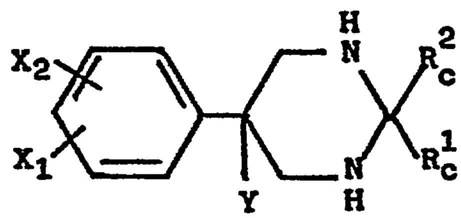

($I_c$)

in welcher $X_1$, $X_2$, Y, $R_c^1$ und $R_c^2$ die obige Bedeutung haben, und von deren Additionssalzen, dadurch gekennzeichnet, daß man 1 Mol 2-Phenyl-1,3-propandiamin der allgemeinen Formel (II) bei 15–25°C unter Atmosphärendruck mit mindestens 1 Mol Carbonylverbindung der allgemeinen Formel ($III_c$ ) in einem geeigneten Lösungsmittel während mindestens 3 Stunden umsetzt.

7. Verfahren nach einem der Ansprüche 1–3 zur Herstellung von Verbindungen der allgemeinen Formel (I) bzw. ($I_a$), in welcher $R_a$ von Wasserstoff verschieden ist, wenn gleichzeitig (i) Y = H, (ii) $X_2$ = H und (iii) $X_1$ = H oder 4–Cl, dadurch gekennzeichnet, daß man die entsprechenden Ausgangsverbindungen einsetzt.

8. Verfahren nach Anspruch 2 oder 7, zur Herstellung von Verbindungen der allgemeinen Formel (I) bzw. ($I_a$), in welcher $X_1$ = 2–F und $X_2$ = Y = $R_a$ = H; in welcher $R_a$ = $COCH_3$ und $X_1$ = $X_2$ = Y = H; und in

welcher Y = OH und $X_1 = X_2 = R_a$ = H, dadurch gekennzeichnet, daß man die entsprechenden Ausgangsverbindungen einsetzt.

9. Verfahren nach Anspruch 4 oder 5 zur Herstellung von Verbindungen der allgemeinen Formel ($I_b$), in welcher $R_b = CH_3$ und $X_1 = X_2$ = Y = H, dadurch gekennzeichnet, daß man die entsprechenden Ausgangsverbindungen einsetzt.

10. Verfahren nach Anspruch 6 zur Herstellung von Verbindungen der allgemeinen Formel ($I_c$), in welcher $X_1$ = 2–F, $X_2$ = Y = H und $R_c^1 = R_c^2 = CH_3$, und in welcher Y = OH, $X_1 = X_2$ = H und $R_c^1 = R_c^2 = CH_3$, dadurch gekennzeichnet, daß man die entsprechenden Ausgangsverbindungen einsetzt.

## Claims for the contracting states AT, ES, GR

1. A method for preparing a 5-phenyl-1,4,5,6-tetrahydropyrimidine derivative selected from the group comprising:
(i) the 5-phenylhydropyrimidines of the formula:

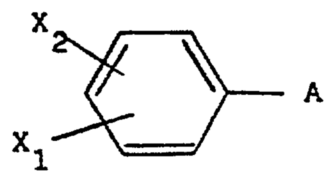

(I)

in which: $X_1$ and $X_2$, which are identical or different, each represent H, F, Cl, Br or $CF_3$, and A represents a polyhydropyrimidinyl group of the structure:

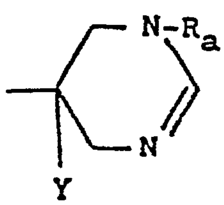 , 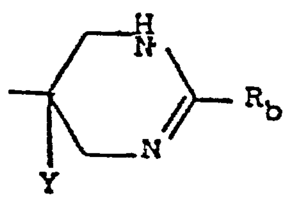 or 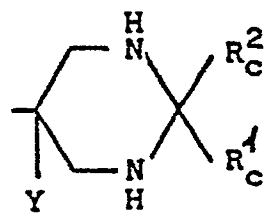

in which: Y represents H or OH, $R_a$ represents a $C_1$–$C_4$ alkyl group or a $C_2$–$C_5$ alkanoyl group, it being possible for $R_a$ to represent the hydrogen atom when at least one of the groups $X_1$, $X_2$ and Y is different from H, $R_b$ represents a $C_1$–$C_3$ alkyl group, $R_c^1$ and $R_c^2$ which are identical or different, each represent the hydrogen atom or a $C_1$–$C_3$ alkyl group; and
(ii) their addition salts,
said method comprising
(a) reacting a 2-phenylpropane-1,3-diamine of the formula:

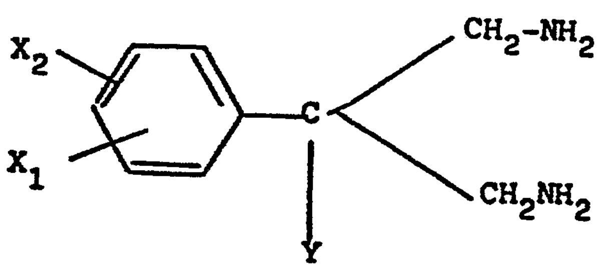

(II)

in which Y, $X_1$ and $X_2$ are defined as indicated above, with a reagent selected from the group comprising:
(i) the alkyl formates of the formula:
H–COO–Alk ($III_a$)
in which Alk represents a $C_1$–$C_3$ lower alkyl group preferably ($CH_2CH_3$), to give, by cyclization, a compound of the formula:

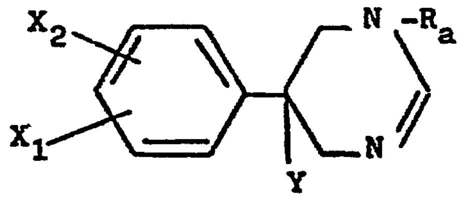

$(I_a)$

in which Y, $X_1$ and $X_2$ are defined as above and $R_a$ is H;
(ii) the alkylamidines of the formula:

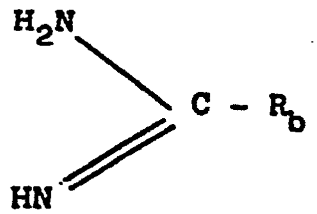

$(III_b)$

in which $R_b$ is defined as indicated above, to give, by cyclization, a compound of the formula:

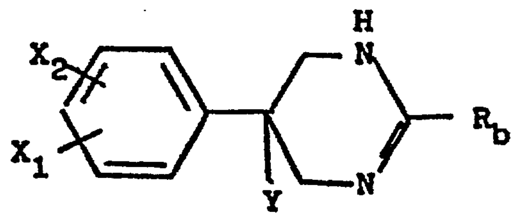

$(I_b)$

in which $X_1$, $X_2$, Y and $R_b$ are defined as above; and
(iii) the aldehydes and ketones of the formula:
$R_c^1 R_c^2 CO$ $(III_c)$
in which $R_c^1$ and $R_c^2$ are defined as indicated above, to give, by cyclization, a compound of the formula:

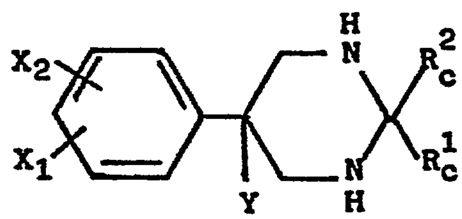

$(I_c)$

in which $X_1$, $X_2$, Y, $R_c^1$ and $R_c^2$ are defined as above, and, if necessary,
(b) reacting the said derivative of the formula $I_a$ in which $R_a$ is H with a reagent selected from the group comprising alkylating agents and acylating agents, to give a compound of the formula $I_a$ in which $R_a$ is a $C_1$–$C_4$ alkyl group or, respectively, a $C_2$–$C_5$ alkanoyl group.
2. A method according to claim 1 for the preparation of compound of the formula

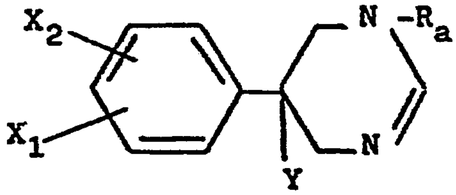

$(I_a)$

wherein $X_1$, $X_2$, Y and $R_a$ are defined as indicated above, and addition salts thereof, said method comprising reacting for at least 2 hours, 1 mol of a 2-phenyl-1,3-propanediamine compound of the formula II with at least 1 mol of an alkyl formate compound of the formula $III_a$, at a temperature higher than or equal to 100°C, and if needed, subjecting the compound of the formula $I_a$ thus obtained in which $R_a$ is H, to a N-alkylating- or N-acylating reaction in order to produce a compound of the formula $I_a$ in which $R_a$ is alkyl or alkanoyl.

3. A method according to claim 2, which comprises reacting at least one mol of $III_a$ with one mol of II, for 2.5–3.5 hours at 100°C under atmospheric pressure, then for 1–5 hours at 100–160°C under reduced pressure.

4. A method according to claim 1, for the preparation of a compound of the formula

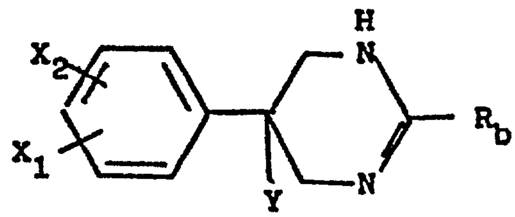

$(I_b)$

wherein $X_1$, $X_2$, Y and $R_b$ are defined as indicated above, and addition salts thereof, said method comprising reacting 1 mol of a 2-phenyl-1,3-propanediamine compound of the formula II with at least 1 mol of an alkylamidine compound of the formula $III_b$, in a suitable solvent, at the reflux temperature of the reaction medium until the evolution of $NH_3$ has ended.

5. A method according to claim 4, which comprises reacting 1 mol of II with 1.5–2.5 mols of $III_b$.

6. A method according to claim 1, for the preparation of a compound of the formula

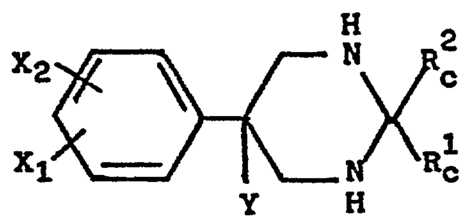

$(I_c)$

wherein $X_1$, $X_2$, Y, $R_c^1$ and $R_c^2$ are defined as indicated above, said method comprising reacting at 15–25°C under atmospheric pressure, 1 mol of a 2-phenyl-1,3-propanediamine compound of the formula II with at least 1 mol of a carbonyl compound of the formula $III_c$, in a suitable solvent, for at least 3 hours.

7. A method according to claim 1, 2 or 3, wherein $R_a$ is different from H when simultaneously (i) Y is H (ii) $X_2$ is H, and (iii) $X_1$ is H or 4–Cl.

8. A method according to claim 2 or 7 wherein (a) $X_1$ = 2–F $X_2$ = Y = $R_a$ = H; (b) $R_a$ = $COCH_3$ and $X_1$ = $X_2$ = Y = H; and (c) Y = OH and $X_1$ = $X_2$ = $R_a$ = H.

9. A method according to claim 4 or 5, wherein $R_b$ = $CH_3$ and $X_1$ = $X_2$ = Y = H.

10. A method according to claim 6, wherein (a) $X_1$ = 2–F, $X_2$ = Y = H and $R_c^1$ = $R_c^2$ = $CH_3$; and (b) Y = OH, $X_1$ = $X_2$ = H and $R_c^1$ = $R_c^2$ = $CH_3$.

**Claims for the contracting states: BE, CH, DE, FR, GB, IT, LI, LU, NL, SE**

1. A 5-phenyl-1,4,5,6-tetrahydropyrimidine derivative selected from the group comprising:
(i) the 5-phenylhydropyrimidines of the formula:

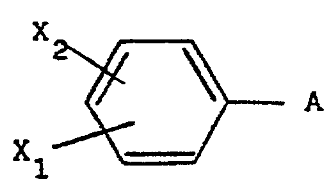

(I)

in which: $X_1$ and $X_2$, which are identical or different, each represent H, F, Cl, Br or $CF_3$, and A represents a polyhydropyrimidinyl group of the structure:

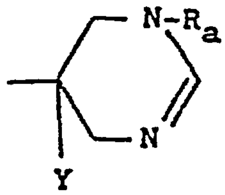 , 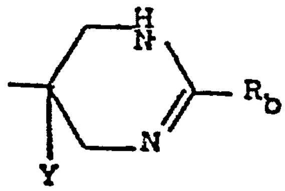 or 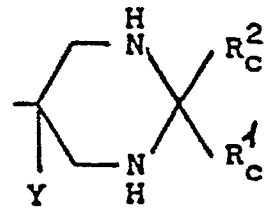

in which: Y represents H or OH, $R_a$ represents a $C_1$–$C_4$ alkyl group or a $C_2$–$C_5$ alkanoyl group, it being possible for $R_a$ to represent the hydrogen atom when at least one of the groups $X_1$, $X_2$ and Y is different from H, $R_b$ represents a $C_1$–$C_3$ alkyl group, and $R_c^1$ and $R_c^2$ which are identical or different, each represent the hydrogen atom or a $C_1$–$C_3$ alkyl group; and
(ii) their addition salts.
2. A derivative according to claim 1 which is selected from the group comprising:
(i) the substituted 5-phenyl-1,4,5,6-tetrahydropyrimidines of the formula:

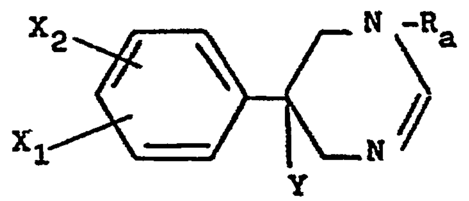 ($I_a$)

in which $X_1$ and $X_2$ which are identical or different, each represent H, F, Cl, Br or $CF_3$, Y is H or OH, and $R_a$ is a $C_1$–$C_4$ alkyl group or a $C_2$–$C_5$ alkanoyl group, it being possible for $R_a$ to represent the hydrogen atom when at least one of the groups $X_1$, $X_2$ and Y is different from H; and
(ii) their addition salts.
3. A derivative according to claim 1 which is selected from the group comprising:
(i) the 2-alkyl-5-phenyl-1,4,5,6-tetrahydropyrimidines of the formula:

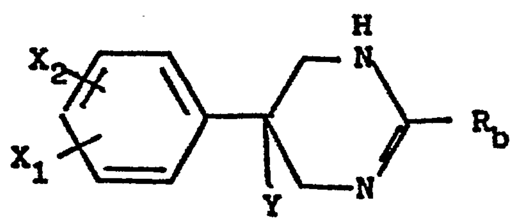 ($I_b$)

in which: $X_1$ and $X_2$, which are identical or different, each represent H, F, Cl, Br or $CF_3$, Y is H or OH, and $R_b$ is a $C_1$–$C_3$ alkyl group; and
(ii) their addition salts.
4. A derivative according to claim 1 which is selected from the group comprising:
(i) the 5-phenyl-1,2,3,4,5,6-hexahydropyrimidines of the formula:

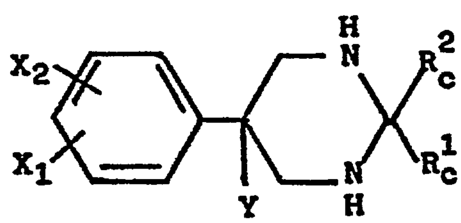 ($I_c$)

in which: $X_1$ and $X_2$, which are identical or different, each represent H, F, Cl, Br or $CF_3$, Y is H or OH, and $R_c^1$ and $R_c^2$ which are identical or different, each represent H or a $C_1$–$C_3$ alkyl group; and
(ii) their addition salts.
5. A derivative according to claim 1 which is selected from the group comprising:
(a) the substituted 5-phenyl-1,4,5,6-tetrahydropyrimidines of the formula:

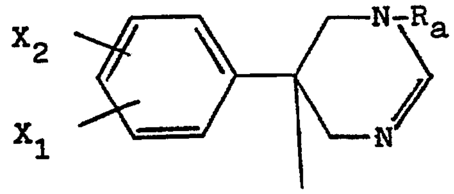

($I'_a$)

in which: $X_1$ and $X_2$, which are identical or different, each represent H, F, Cl, Br or $CF_3$, Y is H or OH, and $R'_a$ is H, $C_1$–$C_4$ alkyl or $C_2$–$C_5$ alkanoyl, $R'_a$ being different from H if, simultaneously (i) Y = H, (ii) $X_2$ = H and (iii) $X_1$ = H or 4–Cl;

(b) the 2-alkyl-5-phenyl-1,4,5,6-tetrahydropyrimidines of the formula:

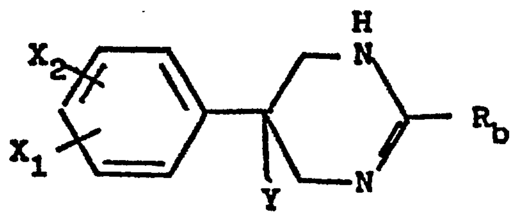

($I_b$)

in which: $X_1$ and $X_2$, which are identical or different, each represent H, F, Cl, Br or $CF_3$, Y is H or OH, and $R_b$ is a $C_1$–$C_3$ alkyl group;

(c) the 5-phenyl-1,2,3,4,5,6-hexahydropyrimidines of the formula:

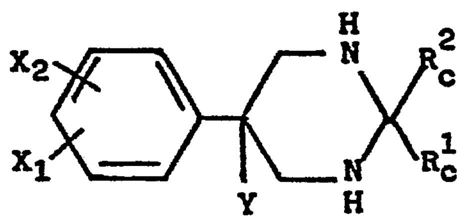

($I_c$)

in which: $X_1$ and $X_2$, which are identical or different, each represent H, F, Cl, Br or $CF_3$, Y is H or OH, and $R^1_c$ and $R^2_c$ which are identical or different, each represent H or a $C_1$–$C_3$ alkyl group; and

(d) their addition salts.

6. 5-(2-Fluorophenyl)-1,4,5,6-tetrahydropyrimidine, and its addition salts.

7. 1-Acetyl-5-phenyl-1,4,5,6-tetrahydropyrimidine, and its addition salts.

8. 5-Hydroxy-5-phenyl-1,4,5,6-tetrahydropyrimidine, and its addition salts.

9. 2-Methyl-5-phenyl-1,4,5,6-tetrahydropyrimidine, and its addition salts.

10. 2,2-Dimethyl-5-hydroxy-5-phenyl-1,2,3,4,5,6-hexahydropyrimidine, and its addition salts.

11. A therapeutic composition comprising, in association with a physiologically acceptable excipient, a pharmaceutically effective amount of at least one derivative of the formula I according to claim 1, or one of its non-toxic addition salts.

12. The use of a substance selected from the group consisting of 5-phenyl-1,4,5,6-tetrahydropyrimidine of the formula $I_a$ according to claim 2 and of the formula $I_b$ according to claim 3, and their non-toxic addition salts, for the preparation of a CNS-antidepressant medicament destined to be used in human therapy against depressions and depressive states.

13. A method for the preparation of a derivative of the formula I according to claim 1, which comprises:

(a) reacting a 2-phenylpropane-1,3-diamine of the formula:

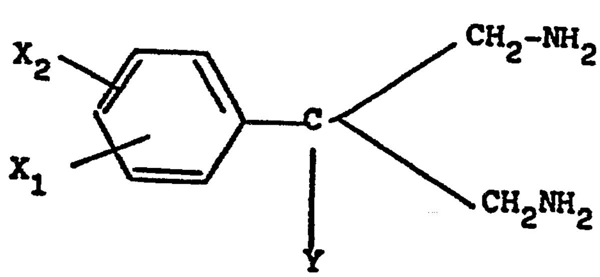

(II)

in which Y, $X_1$ and $X_2$ are defined as indicated above, with a reagent selected from the group comprising:

(i) the alkyl formates of the formula:

H–COO–Alk ($III_a$)

in which Alk represents a $C_1$–$C_3$ lower alkyl group (preferably $CH_2CH_3$), to give, by cyclization, a compound of the formula:

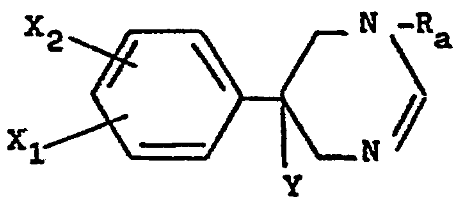

($I_a$)

in which Y, $X_1$ and $X_2$ are defined as above and $R_a$ is H;

(ii) the alkylamidines of the formula:

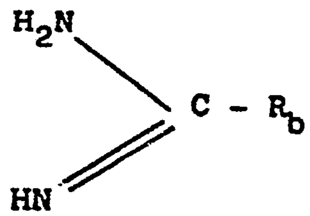

($III_b$)

in which $R_b$ is defined as indicated above, to give, by cyclization, a compound of the formula:

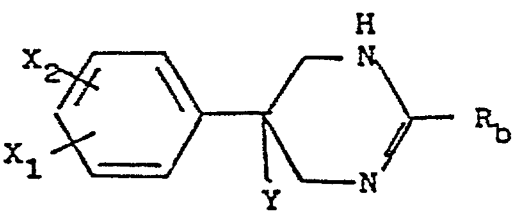

($I_b$)

in which $X_1$, $X_2$, Y and $R_b$ are defined as above; and

(iii) the aldehydes and ketones of the formula:

$R_c^1 R_c^2$ CO ($III_c$)

in which $R_c^1$ and $R_c^2$ are defined as indicated above, to give, by cyclization, a compound of the formula:

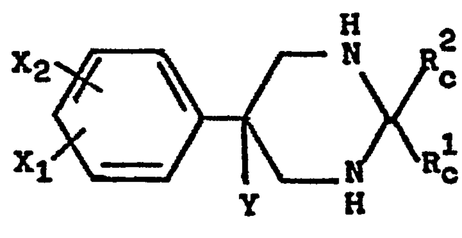

($I_c$)

in which $X_1$, $X_2$, Y, $R_c^1$ and $R_c^2$ are defined as above; and, if necessary,

(b) reacting the said derivative of the formula Ia in which $R_a$ is H with a reagent selected from the group comprising alkylating agents and acylating agents, to give a compound of the formula $I_a$ in which $R_a$ is a $C_1$–$C_4$ alkyl group or, respectively, a $C_2$–$C_5$ alkanoyl group.